# EUROPÄISCHE PATENTSCHRIFT

(11) **EP 0 804 919 B1**
(45) Veröffentlichungstag und Bekanntmachung des Hinweises auf die Patenterteilung: **14.11.2001**
(21) Anmeldenummer: 97250146.4
(22) Anmeldetag: 29.04.1997
(51) Int. Cl.: A61K 6/093

(54) **Verwendung einer Zusammensetzung als Dentalmaterial und Dentalmaterial**
Use of a composition as dental material and dental material
Utilisation d'une composition comme matériau dentaire et matériau dentaire

(30) Priorität: 02.05.1996 DE 19619046
(43) Veröffentlichungstag der Anmeldung: 05.11.1997
(73) Patentinhaber: Ivoclar Vivadent AG, 9494 Schaan (LI)
(72) Erfinder: Rheinberger, Volker, Dr., 9490 Vaduz (LI); Moszner, Norbert, Prof. Dr., 9492 Eschen (LI); Salz, Ulrich, Dr., 88131 Lindau (DE)
(74) Vertreter: UEXKÜLL & STOLBERG

(56) Entgegenhaltungen:
- WO-A-92/16183
- WO-A-93/07230
- DE-A- 2 758 415
- DE-C- 4 416 857
- PATENT ABSTRACTS OF JAPAN vol. 018, no. 361 (C-1222), 7.Juli 1994 & JP 06 093236 A (TOKUYAMA SODA CO LTD), 5.April 1994,

## Beschreibung

Die Erfindung betrifft die Verwendung einer Zusammensetzung als Dentalmaterial sowie ein Dentalmaterial und insbesondere die Verwendung solcher Zusammensetzungen, die ein Kieselsäurekondensat eines hydrolysierbaren und polymerisierbaren Norbornensilans oder ein Kieselsäurekondensat eines hydrolysierbaren und polymerisierbaren Mercaptosilans zusammen mit einem Reaktionspartner für eine Thiol-En-Polymerisation enthalten.

Hydrolysierbare und organisch modifizierte Silane sind bekannt und finden breite Anwendung bei der Herstellung kratzfester Beschichtungen für verschiedene Substrate und bei der Herstellung von Füllstoffen, Klebe- und Dichtungsmassen oder Formkörpern. Dabei werden diese Silane entweder alleine, in Mischungen oder in Gegenwart weiterer hydrolysierbarer und/oder kondensierbarer Komponenten eingesetzt, wobei die Härtung der erhaltenen Hydrolysate oder Kondensate thermisch, photochemisch oder redox-induziert erfolgt.

So beschreibt die DE-C-34 07 087 kratzfeste Beschichtungen, die durch hydrolytische Kondensation einer Mischung aus hydrolysierbaren Titan- oder Zirkoniumverbindungen mit einem hydrolysierbaren Silan R'ₘ(R"Y)ₙSiX₍₄₋ₘ₋ₙ₎ entstehen, wobei R' zum Beispiel Alkyl oder Alkenyl ist, R" zum Beispiel Alkylen oder Alkenylen und X einen hydrolysierbaren Rest darstellen.

Aus der DE-A-35 36 716 sind Klebe- und Dichtungsmassen bekannt, die durch hydrolytische Kondensation von einem oder mehreren Organosilanen mit gegebenenfalls weiteren hydrolysierbaren Silanen erhalten worden sind.

Weiter sind Silane mit reaktiven Doppelbindungen bekannt und im Handel erhältlich, wie zum Beispiel (Meth)acryloyloxysilane der folgenden Formel 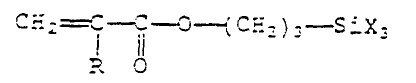 wobei R Wasserstoff oder Methyl bedeutet und X zum Beispiel Halogen oder Alkoxy ist. Diese Silane sind hydrolysierbar und polymerisierbar und können für die Herstellung von Beschichtungen und Klebe- und Dichtungsmassen eingesetzt werden. Infolge des Vorliegens von lediglich einer reaktiven C=C-Doppelbindung bilden diese Silane bei Polymerisation lediglich Kettenpolymere oder sind nach Funktionalisierung durch Reaktion an der C=C-Doppelbindung nicht mehr polymerisierbar. Ferner befindet sich zwischen der Doppelbindung und dem zur Bildung eines anorganischen Netzwerkes befähigten Silicium in der Regel nur eine kurze Kette, so daß die mechanischen Eigenschaften, wie zum Beispiel Flexibilität, über die organischen Gruppen nur in engen Grenzen variiert werden können.

Aus C.J. Brinker, G.W. Scherer, "Sol-Gel-Science", Academic Press, Boston, 1990, Seiten 864 ff., ist es bekannt, daß durch Hydrolyse und Kondensation von vinyl- oder (meth)acrylgruppen-haltigen Silanen oder anderen Metallalkoxiden die "in situ" Bildung von Füllstoffen möglich ist. Die bei der Aushärtung dieser Materialien stattfindenden Polymerisationsreaktionen zum Aufbauen der Polymermatrix werden jedoch mehr oder weniger stark von einem Volumenschrumpf begleitet. Dieser wirkt sich nachteilig auf die Dimensionsstabilität und die mechanischen Eigenschaften von entsprechenden Formkörpern aus. Durch diesen Polymerisationsschrumpf wird ebenso die Haftung von entsprechenden Klebern und die Festigkeit von mit ihrer Hilfe hergestellten Klebeverbindungen nachteilig beeinflußt.

Weitere Beispiele für Kompositmaterialien mit "in situ"-gebildeten Füllstoffteilchen sind aus DE-A-41 33 621 bekannt. Zur Herstellung dieser Materialien werden Sole von zum Beispiel Metalloxiden, wie Titandioxid und Zirkoniumdioxid, stabilisiert und anschließend in ein Polymermatrix kondensiert oder polymerisiert. Zur Bildung der Polymermatrix können verschiedene Monomere und insbesondere solche mit (Meth)acryloyloxy-Resten eingesetzt werden.

Es sind bereits auch eine Reihe von schrumpfungsarmen Matrixsystemen vorgeschlagen worden. Zu deren Herstellung bedient man sich zum Beispiel der doppelten Ringöffnungspolymerisation von verschiedenen cyclischen Monomeren, wie zum Beispiel von Spiroorthoestern oder -carbonaten (vgl. US-A-4 387 215 und R.F. Brady in J. Macromol. Sci.-Rev. Macromol. Chem. Phys. C32, (1992) 135), setzt sogenannte "Low-Profile-Additives" (LPA) ein (vgl. V.A. Pattinson et al. in J. Appl. Polym. Sci. 18, (1974) 2763 und D.-S. Lee et al. in Polym. Eng. Sci. 27, (1987) 964) oder verwendet hochmolekulare Monomere, sogenannte Makromere (vgl. P.F. Rempp et al. in Adv. Polym. Sci. 58, (1984) 1). Alle diese Möglichkeiten haben jedoch bislang keine praktische Bedeutung erlangt, da die Synthese von cyclischen Monomeren aufwendig und teuer ist, der Einsatz von LPA nur auf wenige Fälle, wie zum Beispiel die thermische Härtung ungesättigter Polyesterharze, beschränkt ist und schließlich Makromere nicht nur teuer sind, sondern sich aufgrund ihrer hohen Viskosität nur in geringem Umfang mit Füllstoffen versehen lassen.

Ein relativ geringer Polymerisationsschrumpf tritt teilweise auch bei der aus der US-A-2 347 182 bekannten Polyaddition von Thiolen an C-C-ungesättigte Verbindungen auf. Bei diesem speziellen Polymerisationstyp, der auch als En-Thiol- oder Thiol-En-Polymerisation bezeichnet wird, bilden sich lineare oder vernetzte Polysulfide mit Monoschwefel in der Hauptkette. Die erhaltenen Polysulfide besitzen jedoch Glasübergangstemperaturen, die im allgemeinen unterhalb oder im Bereich der Raumtemperatur liegen (vgl. A.F. Jacobine et al. "Radiation curing of polymeric material", Editor: C.E. Hoyle et al., ACS-Symp. Ser. 417, (1990) 160 und dort zitiert A.F. Jacobine et al. in J. Appl Polym. Sci. 45, (1992) 471). Demzufolge sind mit den herkömmlichen Thiol-En-Polymerisationssystemen nur weiche Materialien mit elastischen oder viskoelastischen Eigenschaften erhältlich.

Der Erfindung liegt nun die Aufgabe zugrunde, die Verwendung einer Zusammensetzung als Dentalmaterial, und insbesondere als dentaler Klebstoff oder Zement oder dentales Füllungsmaterial, zur Verfügung zu stellen, wobei die Zusammensetzung unter Auftreten eines nur geringen Polymerisationsschrumpfes zu Kompositmaterialien mit hoher mechanischer Festigkeit und Härte insbesondere durch Thiol-En-Polymerisation polymerisiert werden kann.

Diese Aufgabe wird durch die Verwendung nach den Ansprüchen 1 bis 17 gelöst.

Die Erfindung betrifft weiter das Dentalmaterial nach Anspruch 18, welches sich durch einen Gehalt an der erfindungsgemäß eingesetzten Zusammensetzung auszeichnet.

Erfindungsgemäß wird eine Zusammensetzung als Dentalmaterial verwendet, welche einen Gehalt an
(a) mindestens einem Kieselsäurekondensat eines hydrolysierbaren und polymerisierbaren Norbornensilans der allgemeinen Formel (Ia) 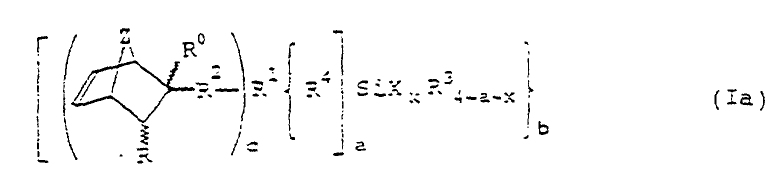 wobei die Variablen R⁰, R, R¹, R², R³, R⁴, R⁵, R⁶, X, Y, Z, a, b, c, x , sofern nicht anders angegeben, unabhängig voneinander die folgenden Bedeutungen haben:
   - R⁰ =: C₁- bis C₈-Alkyl oder H;
   - R =: C₁- bis C₈-Alkyl oder -Alkenyl oder C₆- bis C₁₀-Aryl, wobei diese Reste durch O- oder S-Atome oder durch -O-CO-, -CO-O- oder NH-Gruppen unterbrochen sein können oder diese Atome oder Gruppen endständig tragen können, oder H oder R²-R^{I}-R⁴-SiXₓR³₃₋ₓ;
   - R¹ =: entfällt oder C₁- bis C₈-Alkylen oder C₆- bis C₁₄-Arylen, -Arylenalkylen oder -Alkylenarylen, wobei diese Reste durch O- oder S-Atome oder durch -O-CO-, -CO-O- oder NH-Gruppen unterbrochen sein können oder diese Atome oder Gruppen endständig tragen können;
   - R² =: entfällt oder C₁- bis C₈-Alkylen oder C₆- bis C₁₄-Arylen, -Arylenalkylen oder -Alkylenarylen, wobei diese Reste durch O- oder S-Atome oder durch -O-CO-, -CO-O- oder NH-Gruppen unterbrochen sein können oder diese Atome oder Gruppen endständig tragen können;
   - R³ =: C₁- bis C₁₀-Alkyl oder -Alkenyl oder C₆- bis C₁₀-Aryl, wobei diese Reste durch O- oder S-Atome unterbrochen sein können oder diese Atome endständig tragen können;
   - R⁴ =: -(CHR⁶-CHR⁶)ₙ-, mit n = 0 oder 1, -CHR⁶-CHR⁶-S-R⁵-, -CO-S-R⁵-, -CHR⁶-CHR⁶-NR⁶-R⁵-, -S-R⁵, -Y-CO-NH-R⁵- oder -CO-O-R⁵-;
   - R⁵ =: C₁- bis C₈-Alkylen oder C₆- bis C₁₀-Arylen, wobei diese Reste durch O- oder S-Atome oder durch -O-CO-, -CO-O- oder NH-Gruppen unterbrochen sein können oder diese Atome oder Gruppen endständig tragen können;
   - R⁶ =: H, C₁- bis C₁₀-Alkyl oder C₆- bis C₁₀-Aryl;
   - X =: eine hydrolysierbare Gruppe, nämlich Halogen, OH oder Alkoxy;
   - Y =: O, S oder NR⁶;
   - Z =: O oder CHR⁶;
   - a =: 1, 2 oder 3;
   - b =: 1, 2 oder 3;
   - c =: 1 bis 6; und
   - x =: 1, 2 oder 3;
   und mit der Maßgabe, daß
   - a und/oder b =: 1
   und
   - a+x =: 2, 3 oder 4.
   oder
(b) mindestens einem Kieselsäurekondensat eines hydrolysierbaren und polymerisierbaren Mercaptosilans der allgemeinen Formel (Ib)
   aufweist,

   [(HS-R⁷)_{f}R⁸]_{g}SiXₕR⁹ _{4-g-h} (Ib)

   wobei die Variablen R⁷, R⁸, R⁹, X, f, g und h, sofern nicht anders angegeben, unabhängig voneinander die folgenden Bedeutungen haben:
   - R⁷ =: C₁- bis C₁₀-Alkylen oder -Alkenylen oder C₆-bis C₁₄-Arylen oder -Alkylarylen, wobei diese Reste durch O- oder S-Atome oder -CO-O- oder -O-CO-Gruppen unterbrochen sein können oder diese Atome oder Gruppen endständig tragen können;
   - R⁸ =: C₁- bis C₁₀-Alkylen oder -Alkenylen oder C₆-bis C₁₄-Arylen oder -Alkylarylen, wobei diese Reste durch O- oder S-Atome oder -CO-O- oder -O-CO-Gruppen unterbrochen sein können oder diese Atome oder Gruppen endständig tragen können;
   - R⁹ =: C₁- bis C₁₀-Alkyl oder -Alkenyl oder C₆- bis C₁₄-Aryl- oder -Alkylaryl, wobei diese Reste durch O- oder S-Atome oder -CO-O- oder -O-CO-Gruppen unterbrochen sein können oder diese Atome oder Gruppen endständig tragen können;
   - X =: eine hydrolysierbare Gruppe, nämlich Halogen, Hydroxy oder Alkoxy;
   - g =: 1, 2 oder 3;
   - f =: 1, 2, 3 oder 4; und
   - h =: 1, 2 oder 3.

Dabei decken die obigen Formeln nur solche Verbindungen ab, die mit der Valenzlehre zu vereinbaren sind.

Das erfindungsgemäß eingesetzte Norbornensilan (Ia) liegt üblicherweise in Form von Stereoisomeren-Gemischen, insbesondere als Racemat, vor.

Die bei den vorhandenen Resten möglichen Alkyl-, Alkenyl- und Alkylen-Gruppen können geradkettig, verzweigt oder cyclisch sein. So ist z.B. eine Norbornandiyl-Gruppe eine mögliche C₇-Alkylengruppe.

Weiter können für a, b, c oder x ≥ 1 sowie für f, g oder h ≥ 1 die einzelnen Reste X und die einzelnen R-Reste jeweils unabhängig voneinander gewählt werden.

Außerdem existieren für die oben angegebenen Variablen der Formeln (Ia) und (Ib) bevorzugte Definitionen, die, sofern nicht anders angegeben, unabhängig voneinander gewählt werden können und wie folgt sind:

### Für Formel (Ia):

- R⁰ =: C₁- bis C₅-Alkyl oder H;
- R =: H oder C₁- bis C₅-Alkyl;
- R¹ =: C₁- bis C₈-Alkylen, wobei diese Reste durch O- oder S-Atome oder durch -O-CO- oder -CO-O-Gruppen unterbrochen sein können oder diese Atome oder Gruppen endständig tragen können;
- R² =: C₁- bis C₈-Alkylen, wobei diese Reste durch O- oder S-Atome unterbrochen sein können oder diese Atome endständig tragen können;
- R³ =: CH₃, C₂H₅ oder Phenyl;
- R⁴ =: (CHR⁶-CHR⁶)ₙ-, -S-R⁵, Y-CO-NH-R⁵- oder -CO-O-R⁵-;
- R⁵ =: C₁- bis C₈-Alkylen, wobei diese Reste durch O- oder S-Atome oder durch -O-CO- oder -CO-O-Gruppen unterbrochen sein können oder diese Atome oder Grupen endständig tragen können;
- R⁶ =: H, C₁- bis C₅-Alkyl;
- X =: OCH₃ oder OC₂H₅;
- Y =: O oder S;
- Z =: CH₂;
- a =: 1;
- b =: 1;
- c =: 1 oder 2;
- x =: 2 oder 3;
und/oder
- a+x =: 3 oder 4.

### Für Formel (Ib):

- R⁷ =: C₁- bis C₁₀-Alkylen oder C₆- bis C₁₄-Arylen, wobei diese Reste durch O- oder S-Atome oder -CO-O- oder -O-CO-Grupen unterbrochen sein können oder diese Atome oder Gruppen endständig tragen können;
- R⁸ =: C₁- bis C₁₀-Alkylen oder C₆- bis C₁₄-Arylen, wobei diese Reste durch O- oder S-Atome oder -CO-O- oder -O-CO-Grupen unterbrochen sein können oder diese Atome oder Gruppen endständig tragen können;
- R⁹ =: C₁- bis C₁₀-Alkyl;
- X =: OCH₃, OC₂H₅ oder Cl;
und/oder
- h =: 2 oder 3.

Bevorzugte Ausführungsformen der erfindungsgemäß eingesetzten Norbornensilane haben die nachstehenden allgemeinen Formeln (IIa), (IIIa), (IVa) und (Va), d.h. bei ihnen haben die Indices a und/oder b und/oder c der allgemeinen Formel (Ia) den Wert 1.

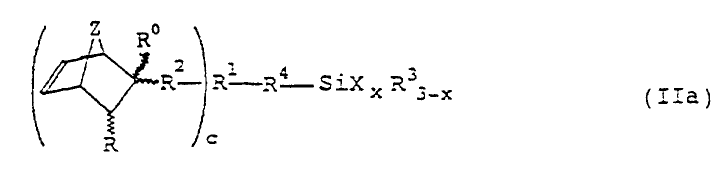

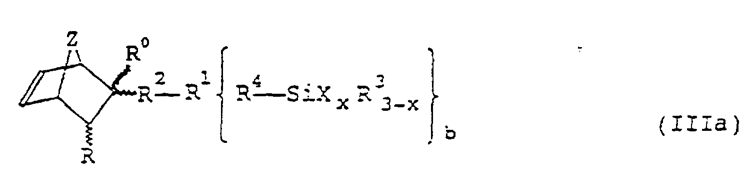

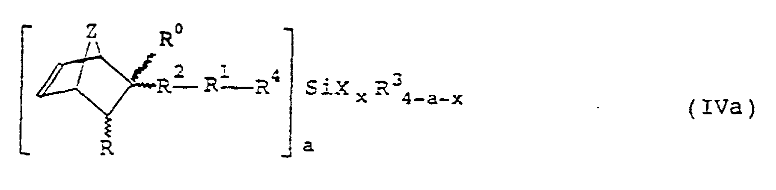

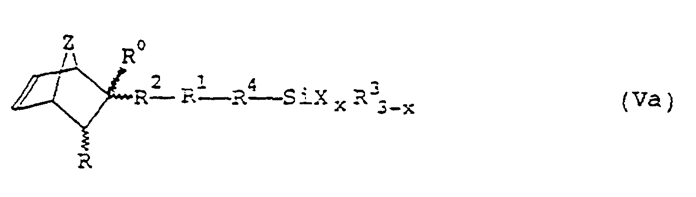

Entsprechendes gilt für die erfindungsgemäß verwendeten Mercaptosilane, und bevorzugte Ausführungsformen für diese Mercaptosilane werden daher durch die nachstehenden allgemeinen Formeln (IIb), (IIIb) und (IVb) dargestellt, bei denen die Indices f und/oder g den Wert 1 haben.

(HS-R⁷)_{f}-R⁸-SiXₕR⁹ ₃₋ₕ (IIb)

HS-R⁷-R⁸-SiXₕR⁹ ₃₋ₕ (IIIb)

[HS-R⁷-R⁸]_{g}SiXₕR⁹ _{4-g-h} (IVb)

Bevorzugte Beispiele für Reste der Norbornensilane mit dem Index a sind im folgenden angegeben. Diese Beispiele gelten auch, soweit die Definition der Mercaptosilane dies zuläßt für die Reste der Mercaptosilane mit dem Index g entsprechend, indem die jeweilige Gruppe 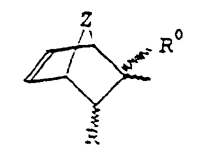 der aufgelisteten Reste von Norbornensilanen durch eine HS-Gruppe ersetzt wird.

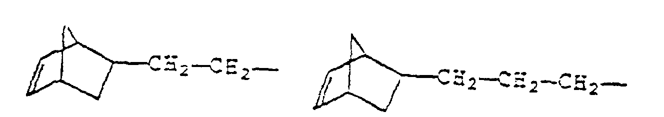

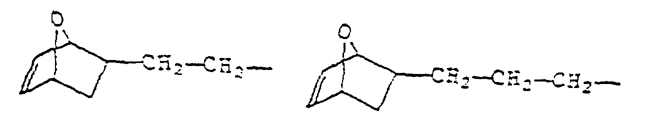

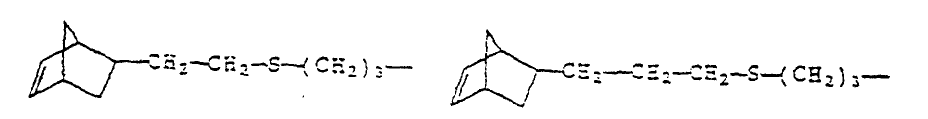

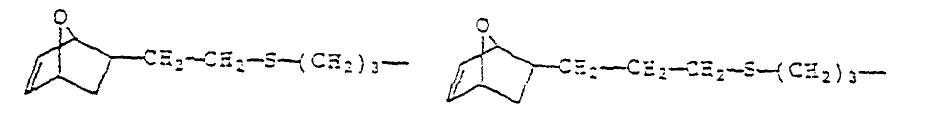

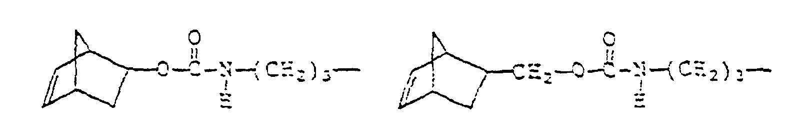

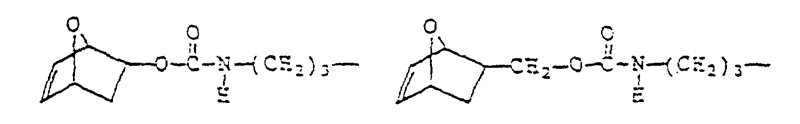

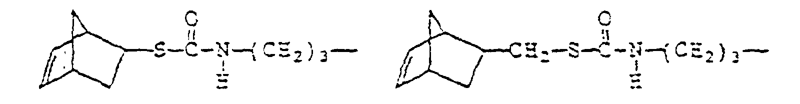

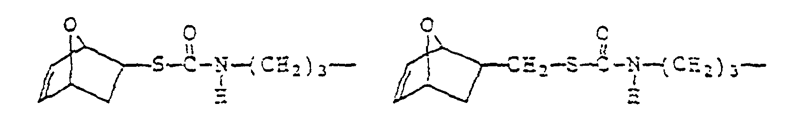

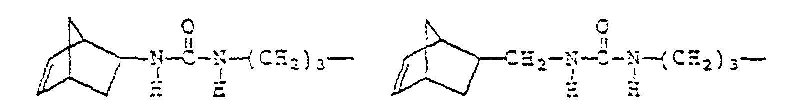

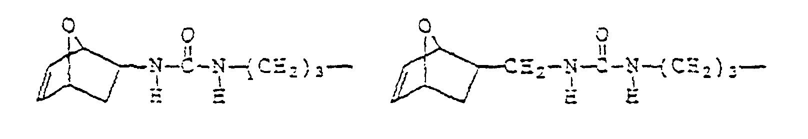

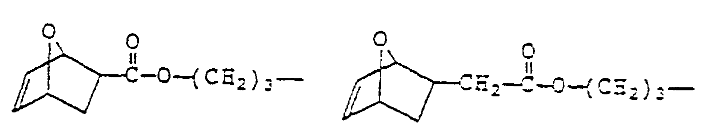

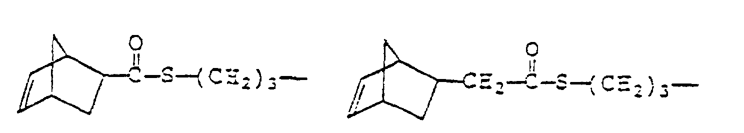

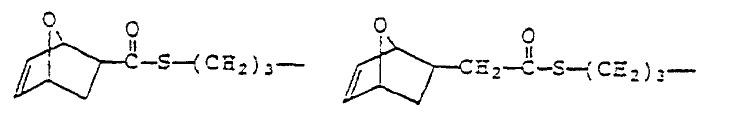

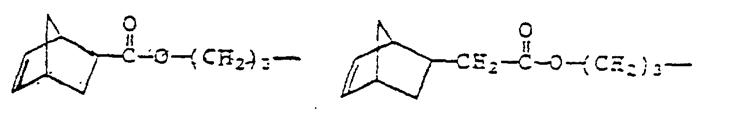

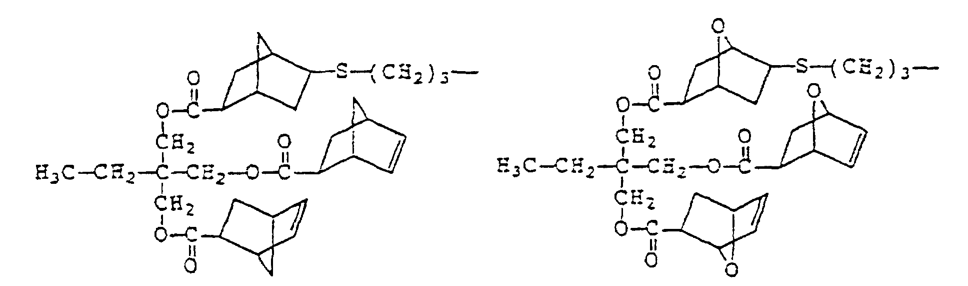

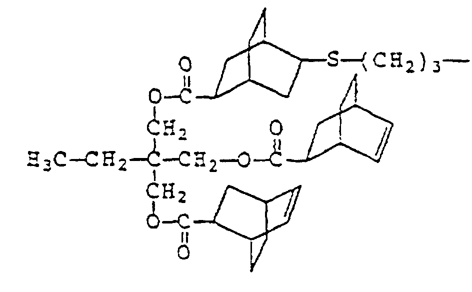

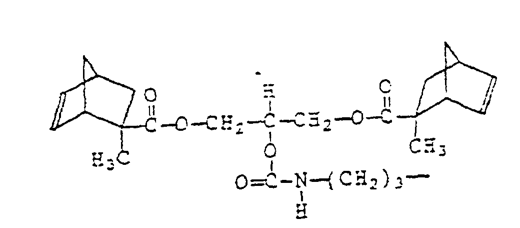

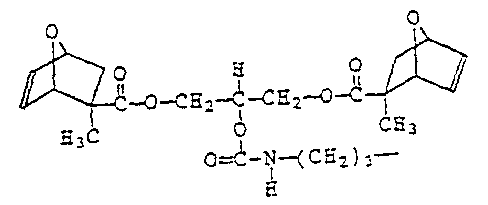

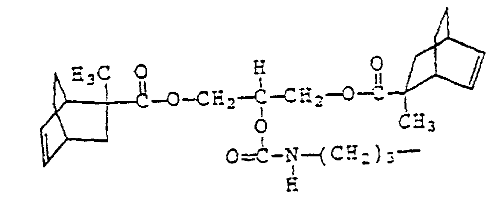

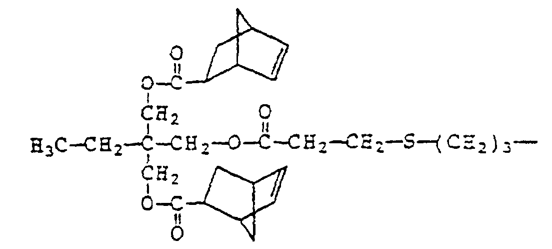

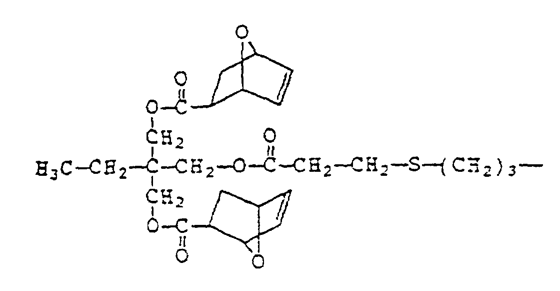

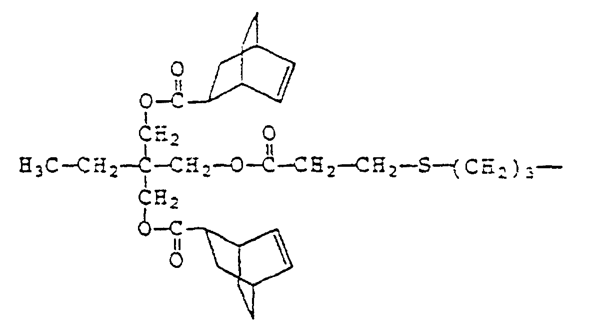

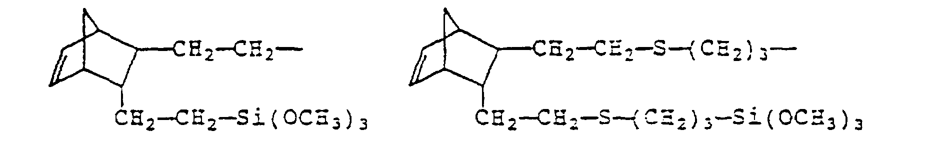

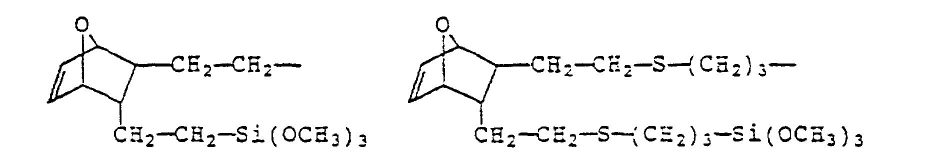

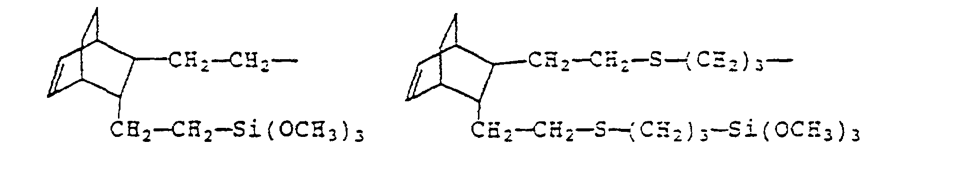

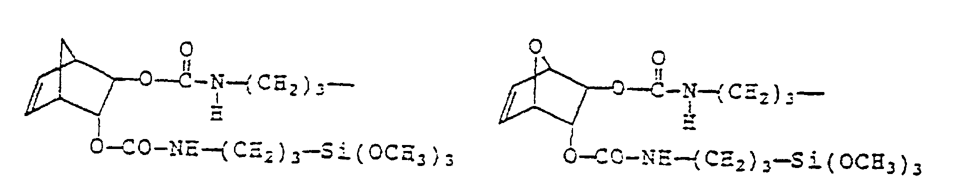

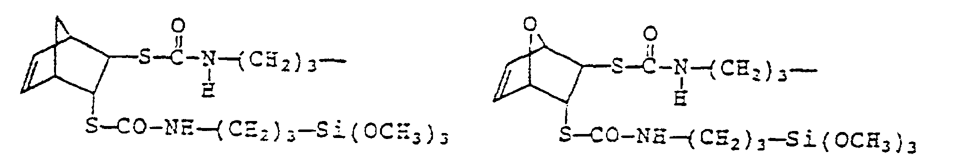

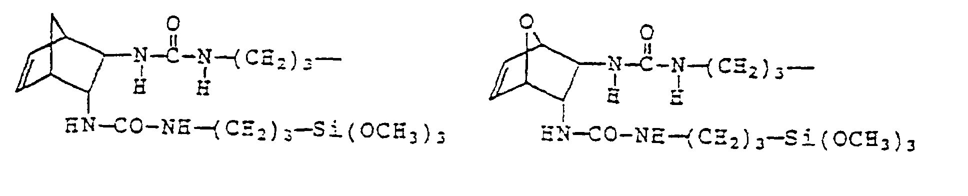

Besonders bevorzugte Mercaptosilane (Ib) sind nachstehend angegeben: 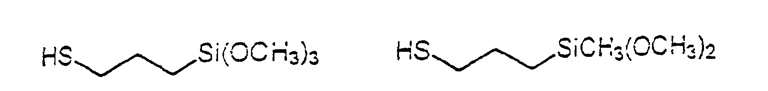 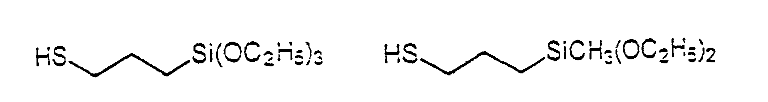 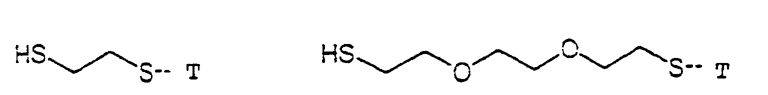 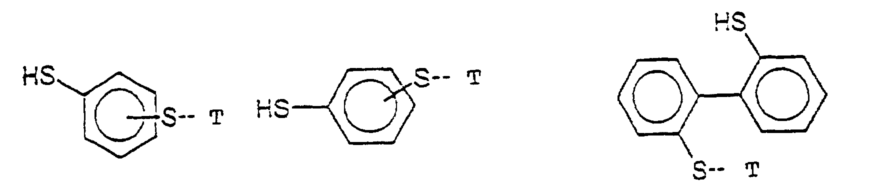 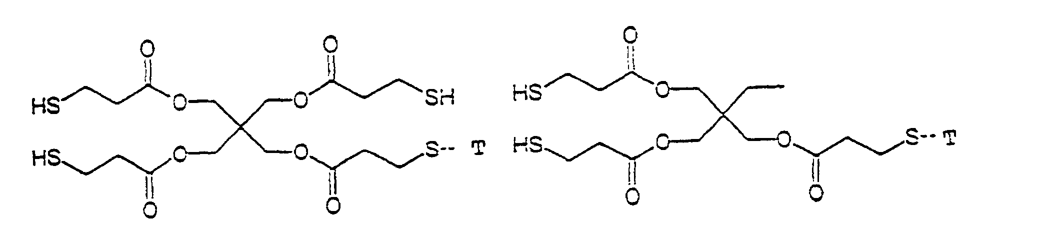 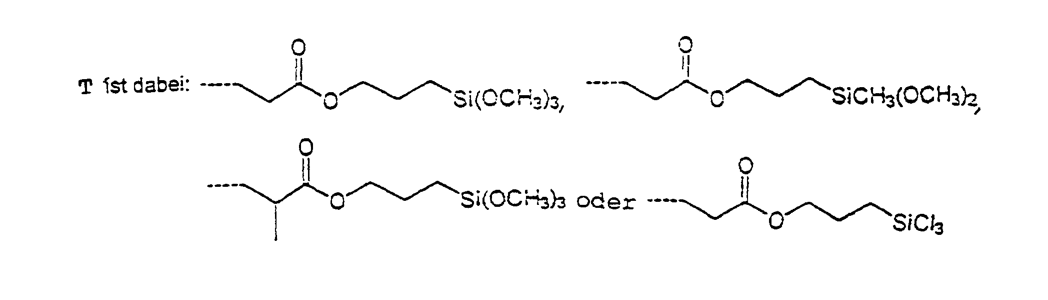 Die Herstellung der erfindungsgemäß eingesetzten Norbornensilane (Ia) ist über eine große Anzahl von Additions- und Kondensationsreaktionen möglich, die nach den für diese Reaktionstypen üblichen Methoden durchgeführt werden.

In einer ersten Variante werden Norbornene oder Oxabicycloheptene, die endständige, olefinische C=C-Doppelbindungen aufweisen, einer Hydrosilylierung, einer Thiol- oder einer Amin-Addition unterzogen. Die allgemeinen Reaktionsschemata sehen wie folgt aus, wobei die Reste und Indices wie im Falle der allgemeinen Formel (Ia) definiert sind. Zur Verdeutlichung sind ebenfalls einfache, konkrete Beispiele angegeben.

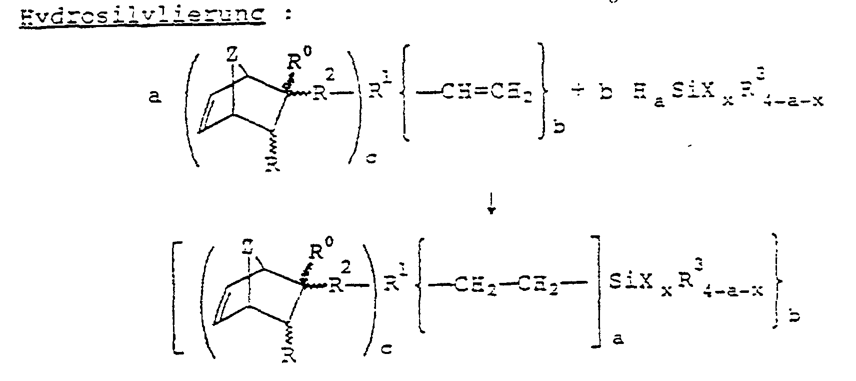

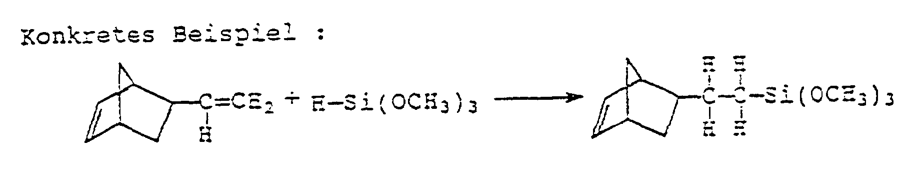

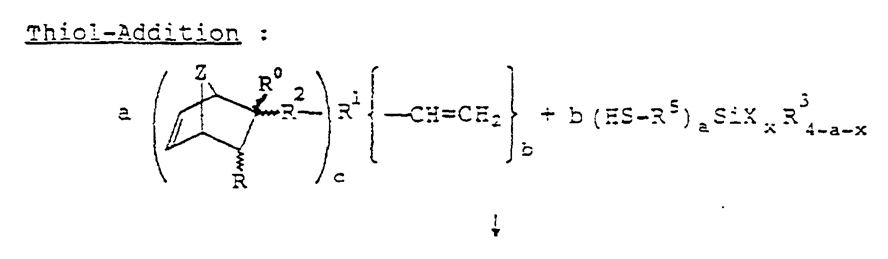

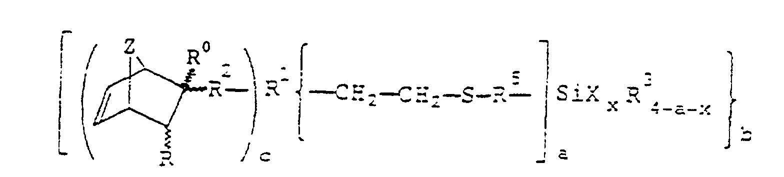

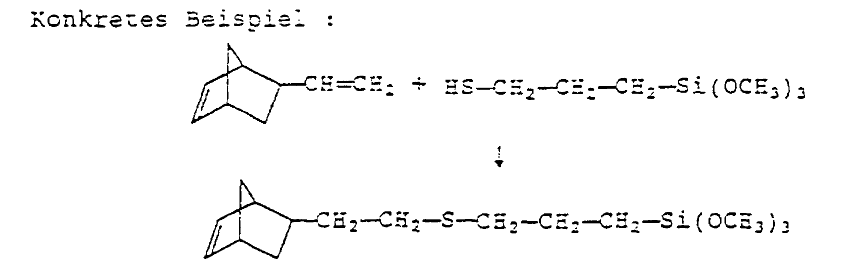

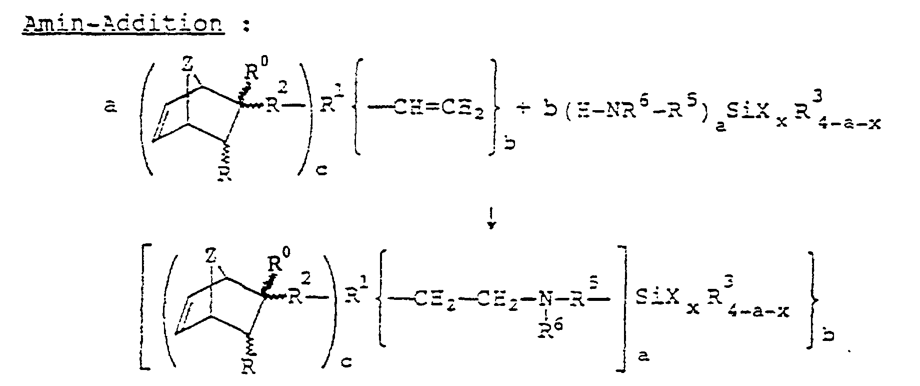

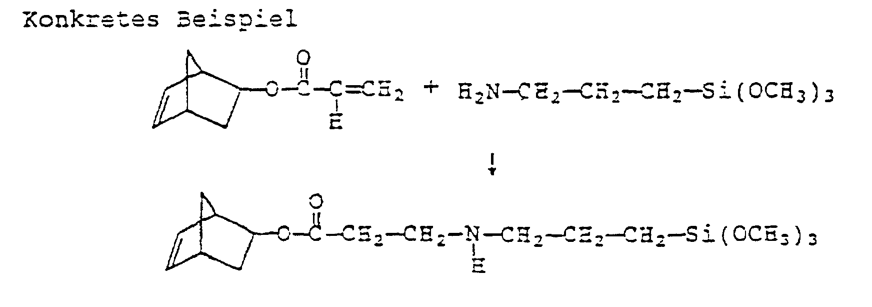

Die Reste R¹ können bis zu b endständige C=C-Doppelbindungen enthalten, so daß jeweils bis zu b Silaneinheiten an den Rest R¹ addierbar sind. Die eingesetzten Silane können bis zu a Wasserstoffatome, Thiol- oder Aminogruppen enthalten, so daß jeweils an ein Silan bis zu a bicyclische Reste addierbar sind. Ferner können die Reste R¹ jeweils bis zu c Norbornen- oder Oxabicyclohepteneinheiten enthalten. Ganz analog werden auch Norbornene bzw. Oxabicycloheptene der allgemeinen Formel XV umgesetzt.

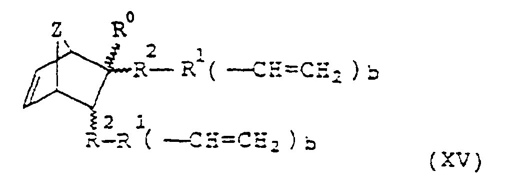

Ferner ist die Hydrosilylierung, die Thiol- oder die Aminaddition auch an den C=C-Doppelbindungen von Norbornenen und Oxabicycloheptenen möglich, falls R¹ über mindestens 2 bicyclische Reste verfügt.

In einer weiteren Verfahrensvariante werden bicyclische Verbindungen, die Hydroxyl-, Thiol- oder Aminogruppen enthalten, an Silane addiert, die Isocyanatfunktionen aufweisen. Die allgemeinen Reaktionsschemata sehen wie folgt aus, wobei die Reste und Indices wie im Falle der allgemeinen Formel (Ia) definiert sind. Zur Veranschaulichung sind ebenfalls einfache, konkrete Beispiele angegeben.

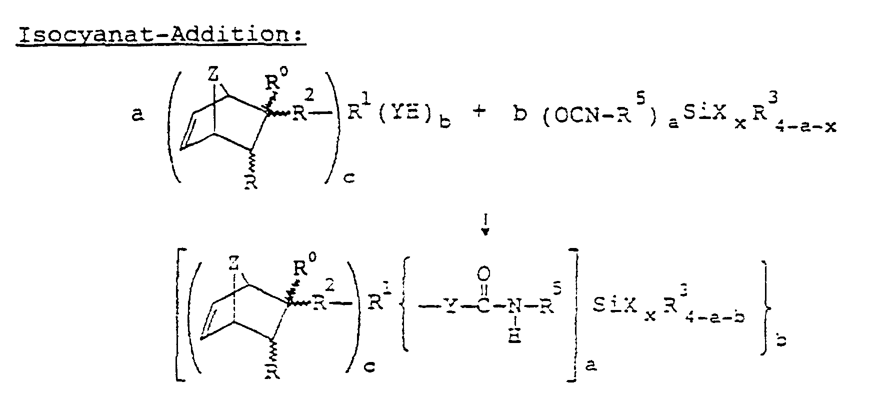

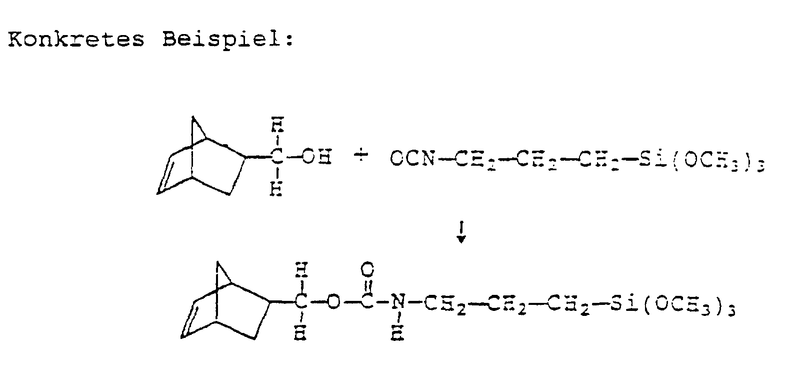

Die Reste R¹ der bicyclischen Komponenten können jeweils bis zu b Hydroxyl-, Thiol- oder Aminogruppen enthalten, so daß jeweils bis zu b Silaneinheiten an den Rest R¹ addierbar sind. Die eingesetzten Silane können jeweils bis zu a Isocyanat-Gruppen aufweisen, so daß an ein Silan bis zu a bicyclische Reste addierbar sind. Ferner kann der Rest R¹ bis zu c Norbornen- oder Oxabicyclohepteneinheiten enthalten. Ganz analog werden auch Norbornene bzw. Oxabicycloheptene der allgemeinen Formel (XVI) umgesetzt.

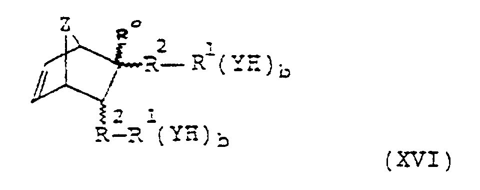

In weiteren Verfahrensvarianten werden bicyclische Carbonsäurederivate mit thiol- oder hydroxylgruppen-haltigen Silanen umgesetzt. Die allgemeinen Reaktionsschemata sind im folgenden dargestellt, wobei -A für -OH, -Cl, -H oder -O-Alkyl steht und die übrigen Reste und Indices wie im Falle der allgemeinen Formel (Ia) definiert sind. Zur Veranschaulichung sind wiederum einfache, konkrete Beispiele ebenfalls angegeben.

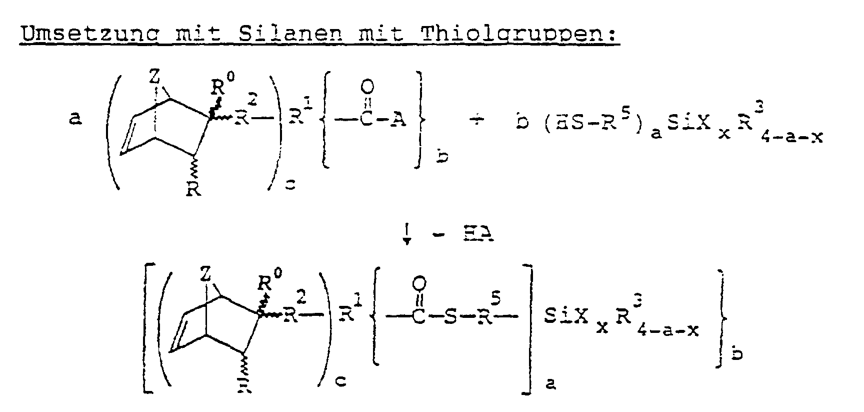

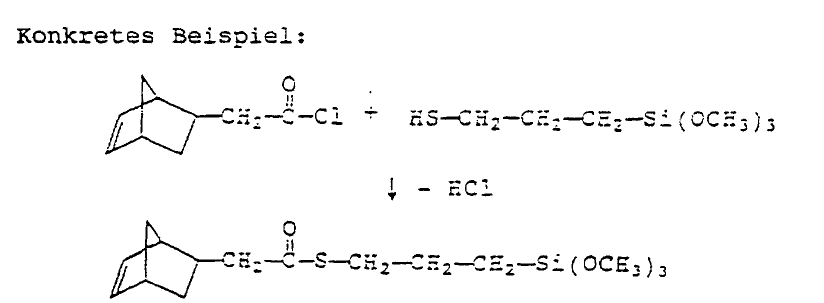

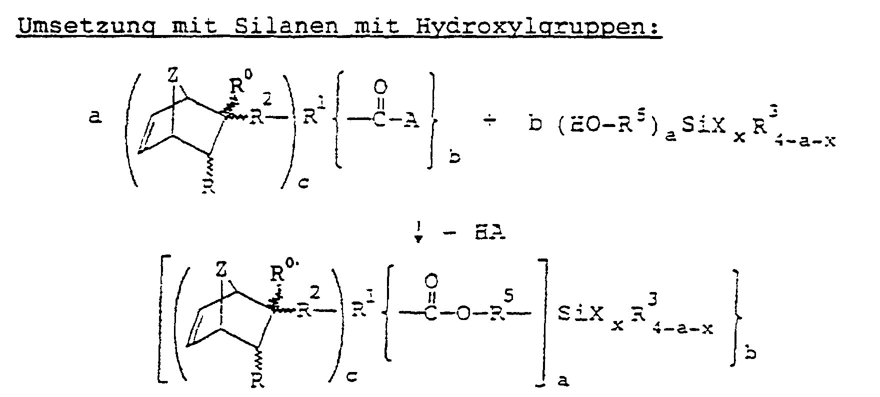

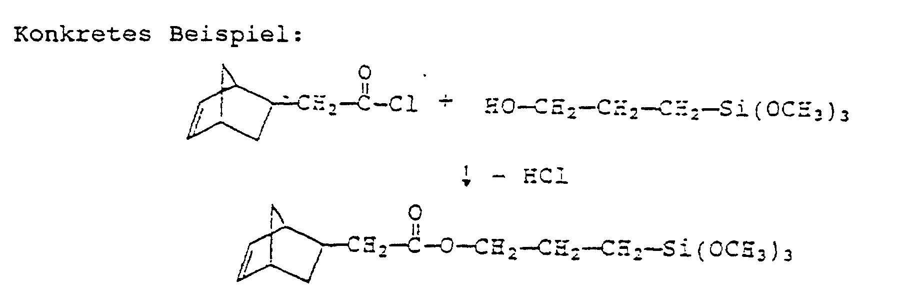

Die Reste R¹ der bicyclischen Komponenten können jeweils bis zu b Carbonsäurederivate enthalten, so daß jeweils bis zu b Silaneinheiten an den Rest R¹ addierbar sind. Die eingesetzten Silane können jeweils bis zu a Thiol- oder Hydroxylgruppen aufweisen, so daß an ein Silan bis zu a bicyclische Reste addierbar sind. Ferner kann der Rest R¹ bis zu c Norbornen- oder Oxabicyclohepteneinheiten enthalten. Ganz analog werden auch Norbornene bzw. Oxabicycloheptene der allgemeinen Formel (XVII) umgesetzt.

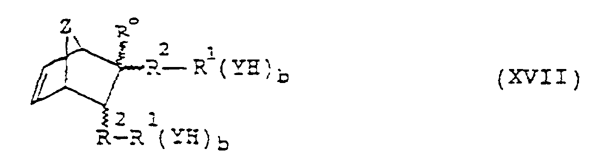

Folgende Additionen sind ebenfalls möglich, wobei die Reste und Indices wie in der allgemeinen Formel (Ia) definiert sind und der Index a bevorzugt > 1 ist:

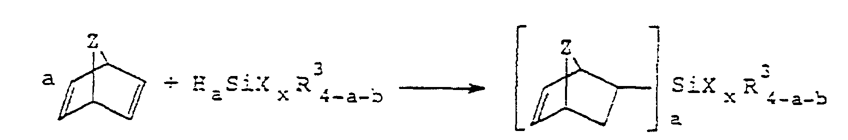

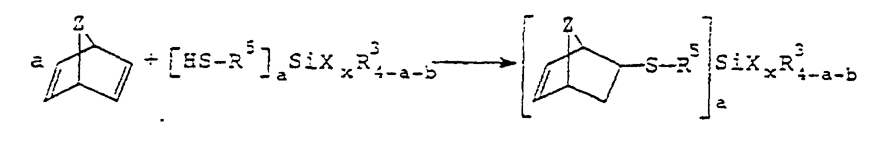

Weiter kann die Norborneneinheit über eine Diels-Alder-Addition eines Furan- oder Cyclopentadien-Derivates an ein organisch modifiziertes Silan, dessen organische(r) Rest(e) eine oder mehrere C=C-Doppelbindungen aufweist, aufgebaut werden. Die allgemeinen Reaktionsschemata sehen wie folgt aus, wobei die Reste und Indices wie im Falle der allgemeinen Formel (Ia) definiert sind.

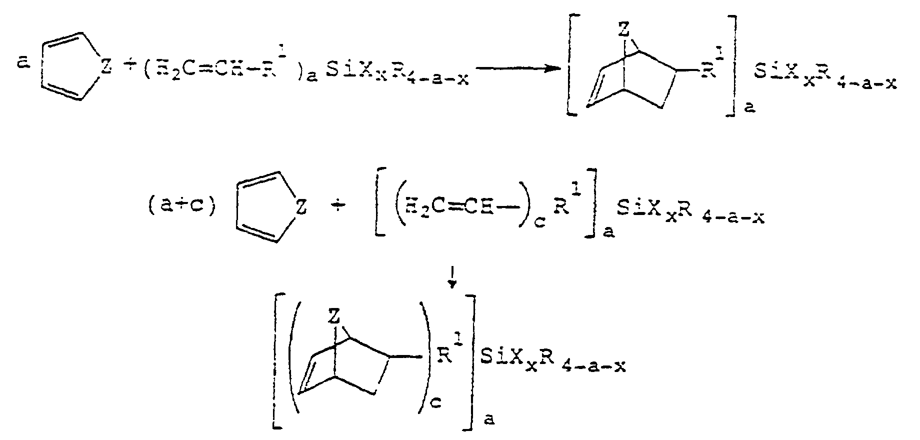

Enthält der Rest R¹ mehr als eine endständige C=C-Doppelbindung, so sind mehr als eine Furan- oder Cyclopentadien-Einheit addierbar.

Die bei den zuvor beschriebenen Beispielen eingesetzten Silane sind käuflich erwerbbar oder nach Methoden herstellbar, wie sie z.B. in "Chemie und Technologie der Silicone" (W. Noll, Verlag Chemie GmbH, Weinheim/Bergstraße, 1968), in DE-C-40 11 044 oder in DE 44 16 857 beschrieben sind.

Die eingesetzten Norbornen- oder Oxabicycloheptenderivate sind z.B. durch übliche Funktionalisierung von Norbornadien- und Oxabicycloheptadienderivaten erhältlich oder durch Diels-Alder-Addition von Furan- bzw. Cyclopentadien-Derivaten an C=C-Doppelbindungen. Ausgangskomponenten der Formel (XVIII), in der die Reste und Indices wie im Falle der allgemeinen Formel (Ia) definiert sind, 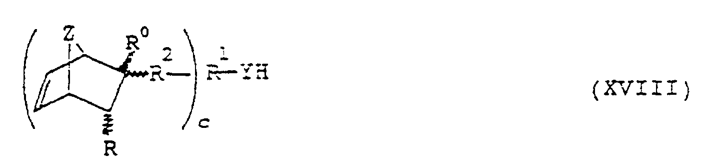 sind z.B. gemäß nachstehender Reaktionsfolge erhältlich.

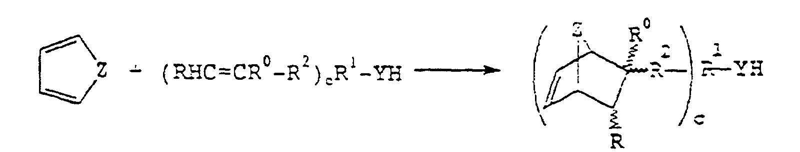

Im folgenden sind weitere konkrete Beispiele zur Herstellung erfindungsgemäß einsetzbarer Norbornensilanen (Ia) mit einer oder mehreren Norbornen-Einheiten angegeben.

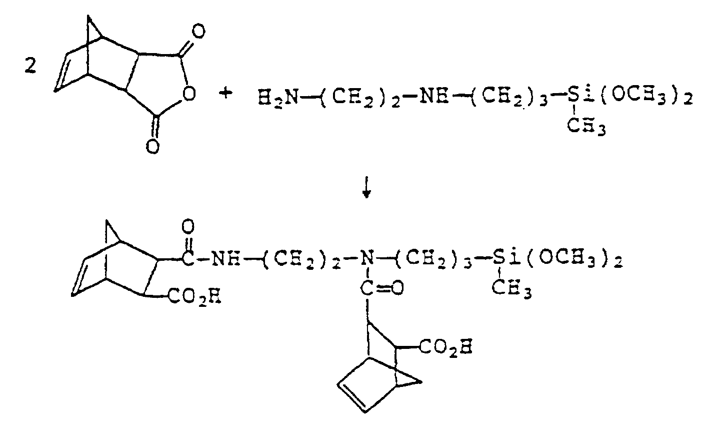

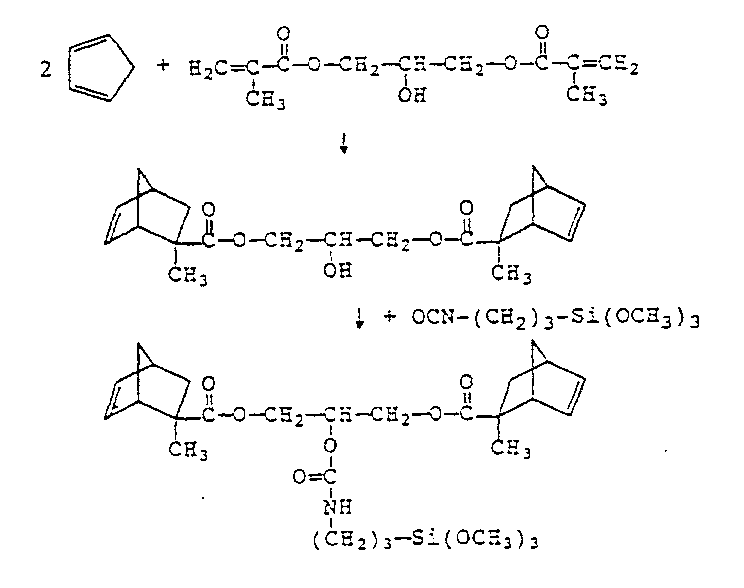

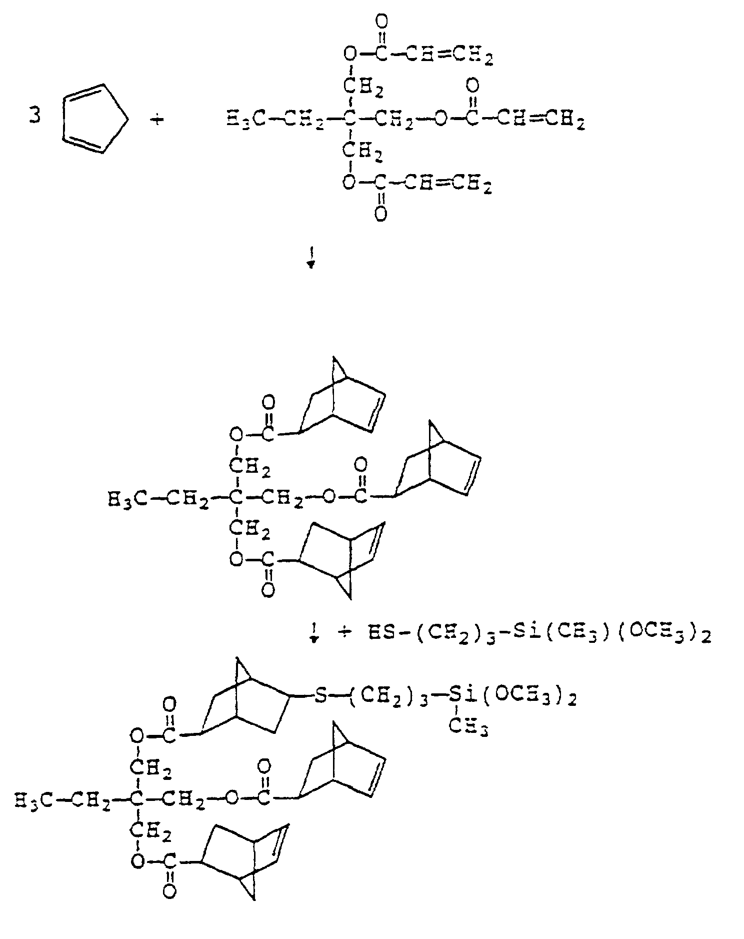

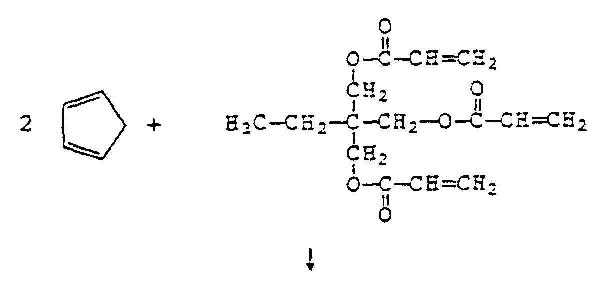

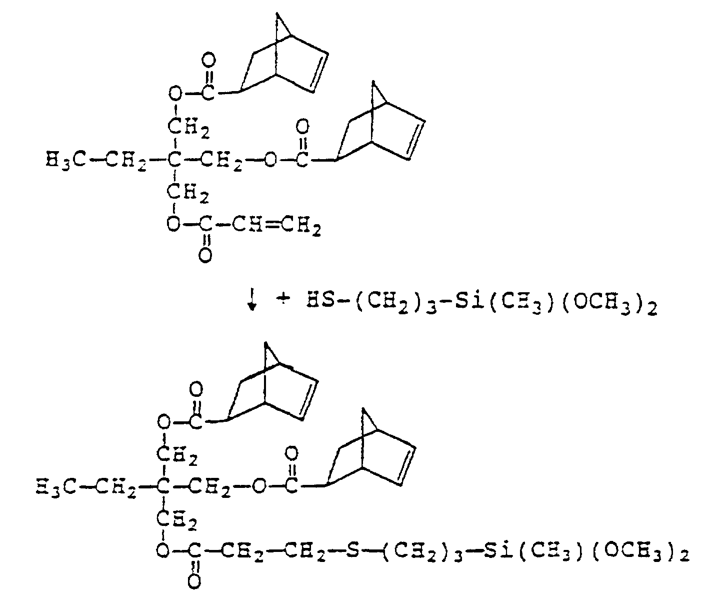

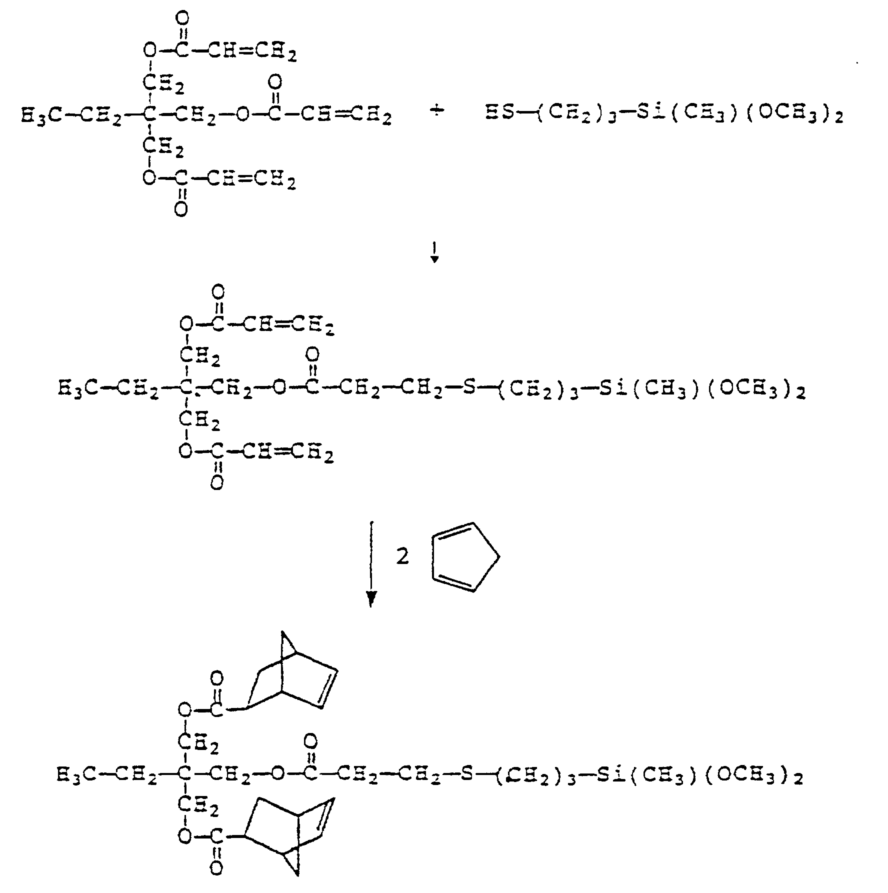

Die erfindungsgemäß eingesetzten Mercatptosilane sind kommerziell zugänglich oder lassen sich nach verschiedenen bekannten Methoden herstellen. Eine Variante ist dabei die partielle SH-En-Addition von multifunktionellen Mercaptoverbindungen an käufliche Acryloylsilane gemäß nachstehender allgemeiner Reaktionsgleichung, wobei die Reste und Indices so wie bei Formel (Ib) definiert sind.

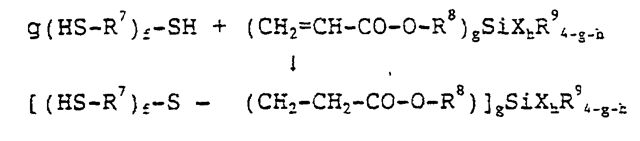

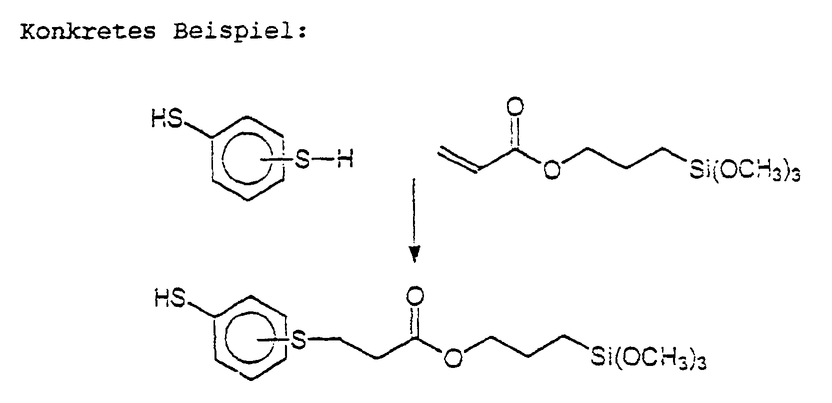

Die erfindungsgemäß eingesetzten Kieselsäurekondensate der Norbornensilane (Ia) und Mercaptosilane (Ib) werden durch Hydrolyse der in den Silanen (Ia) und (Ib) enthaltenen hydrolysierbaren Gruppen X, z.B. Alkoxy-Gruppen, und anschließende Kondensation, welche zur Ausbildung eines anorganischen Netzwerkes aus Si-O-Si-Einheiten führt, erhalten. Die Hydrolyse und Kondensation erfolgt im basischen oder sauren Milieu, wobei eine Verknüpfung von C=C-Doppelbindungen, die in den eingesetzten Silanen enthalten sind, nicht erfolgt. Vorzugsweise erfolgt die Hydrolyse und Kondensation in Gegenwart eines Kondensationskatalysators, wobei protonen- oder hydroxylionen-abspaltende Verbindungen, wie organische oder anorganische Säuren oder Basen, bevorzugt sind. Besonders bevorzugt sind flüchtige Säuren oder Basen, insbesondere Salzsäure oder Ammoniak. Es hat sich bewährt, bei der Hydrolyse und Kondensation Verfahrensweisen der Sol-Gel-Technologie zu übernehmen, wie sie z.B. in C.J. Brinker et al., "Sol-Gel-Science", Academic Press, Boston, 1990, beschrieben sind. Das "Sol-Gel-Verfahren" ist darüber hinaus in DE-A-27 58 414, DE-A-27 58 415, DE-A-30 11 761, DE-A-38 26 715 und DE-A-38 35 968 beschrieben.

Besonders bevorzugt ist es, die für eine vollständige Hydrolyse aller hydrolysierbaren Gruppen stöchiometrisch erforderliche Wassermenge in Form von wasserhaltigen Alkoholen oder feuchtigkeitsbeladenen Adsorbentien hinzuzugeben und die Hydrolyse und Kondensation in einer Lösung von aliphatischen Alkoholen und Estern bei Temperaturen im Bereich von vorzugsweise -5 bis + 30°C durchzuführen.

Die erfindungsgemäß eingesetzten Kieselsäurekondensate des Norbornensilans (Ia) und Mercaptosilans (Ib) können jedoch auch in nicht vollständig hydrolysierter und kondensierter Form eingesetzt werden. In solchen Fällen spricht man auch von sogenannten Vorkondensaten.

Die erfindungsgemäß eingesetzten Zusammensetzungen können neben mindestens einem Kieselsäurekondensat des Norbornensilans (Ia) oder mindestens einem Kieselsäurekondensats des Mercaptosilans (Ib) auch noch weitere hydrolytisch kondensierbare Verbindungen enthalten, die als solche oder in Form von Vorkondensaten oder vollständig hydrolysierten und kondensierten Produkten eingesetzt werden.

Als weitere hydrolytisch kondensierbare Verbindungen wird bevorzugt mindestens ein Silan der allgemeinen Formel (VIII) eingesetzt

R¹⁰ ₖ(Z'R¹¹)ₘSiX'₄₋₍ₖ₊ₘ₎ (VIII)

wobei R¹⁰, Z',R¹¹, X', k und m, sofern nicht anders angegeben, unabhängig voneinander die folgenden Bedeutungen haben:
- R¹⁰ =: C₁- bis C₈-Alkyl, C₁- bis C₁₂-Alkenyl oder C₆- bis C₁₄-Aryl,
- R¹¹ =: C₁- bis C₈-Alkylen, C₁- bis C₁₂-Alkenylen oder C₆-bis C₁₄-Arylen,
- X' =: H, Halogen, OH, C₁- bis C₈-Alkoxy,
- Z' =: Mercapto-, Carboxy-, Acryl-, Methacryl-, Allyl-, Vinyl- oder Vinylether-Gruppe;
- k =: 0, 1, 2 oder 3,
- m =: 0, 1, 2 oder 3, und
- k+m =: 1, 2 oder 3.

Derartige Silane sind zum Beispiel in der DE-C-34 07 087 beschrieben.

Spezielle Beispiele für hydrolytisch kondensierbare Silane der allgemeinen Formel (VIII) sind:

CH₃-Si-Cl₃, CH₃-Si-(OC₂H₅)₃, C₂H₅-Si-Cl₃, C₂H₅-Si-(OC₂H₅)₃, CH₂=CH-Si-(OC₂H₅)₃, CH₂=CH-Si-(OC₂H₄OCH₃)₃, (CH₃)₂-Si-Cl₂, CH₂=CH-Si-(OOCCH₃)₃, (CH₃)₂-Si-(OC₂H₅)₂, (C₂H₅)₃-Si-Cl, (C₂H₅)₂-Si-(OC₂H₅)₂, (CH₃)₂(CH₂=CH)-Si-Cl₂, (CH₃)₃-Si-Cl, (t-C₄H₉)(CH₃)₂-Si-Cl, (CH₃O)₃-Si-C₃H₆-NH-C₂H₄-N-C₂H₄-NH₂, (CH₃O)₃-Si-C₃H₆-SH, (CH₃O)₃-Si-C₃H₆-NH-C₂H₄-NH₂, (CH₃O)₃Si-C₃H₆-Cl, (CH₃O)₃-Si-C₃H₆-O-C(O)-C(CH₃)=CH₂, (CH₃)₂(CH₂=CH-CH₂)-Si-Cl, (C₂H₅O)₃-Si-C₃H₆-NH₂, (C₂H₅O)₃-Si-C₃H₆-CN,

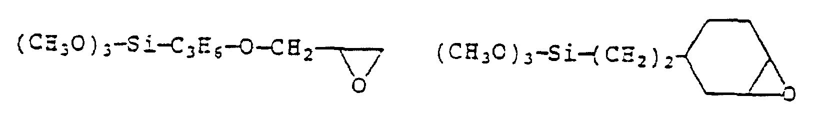

Darüber hinaus können auch als bevorzugte weitere hydrolytisch kondensierbare Verbindungen mindestens eine Aluminium-, Titan- oder Zirkonium-Verbindung der Formel

AlR¹² ₃ oder M X"_{y} R¹³ _{z}

eingesetzt werden, wobei M, R¹², R¹³, X", y und z unabhängig voneinander die folgenden Bedeutungen haben:
- M =: Ti oder Zr,
- R¹² =: Halogen, OH, C₁- bis C₈-Alkoxy,
- R¹³ =: R, wie in Anspruch 1 definiert,
- X" =: Halogen, OH oder C₁- bis C₈-Alkoxy,
- y =: 1 bis 4, insbesondere 2 bis 4,
- z =: 0 bis 3, insbesondere 0 bis 2.

Bevorzugte Beispiele für die einsetzbaren Aluminium-Verbindungen sind Al(OCH₃)₃, Al(OC₂H₅)₃, Al(O-n-C₃H₇)₃, Al(O-i-C₃H₇)₃, Al(OC₄H₉)₃, Al(O-i-C₄H₉)₃, AlCl₃ und AlCl(OH)₂. Bei Raumtemperatur flüssige Verbindungen, wie z.B. Aluminium-sek-butylat und Aluminium-isopropylat, sind besonders bevorzugt.

Bevorzugte Beispiele für die einsetzbaren Titan- und ZirkoniumVerbindungen sind TiCl₄, Ti(OC₂H₅)₄, Ti(OC₃H₇)₄, Ti(O-C₄H₉)₄, Ti(2-ethylhexoxy)₄, ZrCl₄, Zr(OC₂H₅)₄, Zr(OC₃H₇)₄, Zr(OC₄H₉)₄, Zr(2-ethylhexoxy)₄ und ZrOCl₂.

Es ist besonders bevorzugt, daß das erfindungsgemäß eingesetzte Kieselsäurekondensat des Norbornensilans (Ia) oder das Kieselsäurekondensat des Mercaptosilans (Ib) und gegebenenfalls weitere vorhandene hydrolytisch kondensierbare Verbindungen gemeinsam der Hydrolyse und Kondensation unterworfen werden und daher als gemischte Vorkondensate oder gemischte Kondensate eingesetzt werden.

Erhaltene Kieselsäurekondensate von den erfindungsgemäß eingesetzten Silanen (Ia) und (Ib) und gegebenenfalls weiteren hydrolysierbaren und kondensierbaren Verbindungen können entweder als solche oder nach teilweiser oder nahezu vollständiger Entfernung des verwendeten Lösungsmittels eingesetzt werden. In einigen Fällen kann es sich als vorteilhaft erweisen, in dem nach der Polykondensation erhaltenen Produkt Wasser und gegebenenfalls eingesetztes Lösungsmittel durch ein anderes Lösungsmittel zu ersetzen, um das Kieselsäurekondensat zu stabilisieren. Zu diesem Zweck kann die Reaktionsmischung, z.B. im Vakuum bei leicht erhöhter Temperatur, soweit eingedickt werden, daß sie noch problemlos mit einem anderen Lösungsmittel aufgenommen werden kann.

Die Silane (Ia) und (Ib) sind stabile Verbindungen, und sie können entweder alleine oder zusammen mit anderen hydrolysierbaren, kondensierbaren und/oder polymerisierbaren oder polyaddierbaren Komponenten zu den erfindungsgemäß eingesetzten Kieselsäurepolykondensaten und Kieselsäureheteropolykondensaten verarbeitet werden.

Es ist bevorzugt, daß das Kieselsäurekondensat des Norbornensilans (Ia) und des Mercaptosilans (Ib) zusammen mit einem Reaktionspartner für eine Thiol-En-Polymerisation in der erfindungsgemäßen Zusammensetzung enthalten sind.

Bevorzugte Reaktionspartner des Kieselsäurekondensats des Norbornensilans (Ia) sind dabei das Mercaptosilan (Ib), ein Kieselsäurekondensat des Mercaptosilans (Ib) und/oder eine Oligo- oder Poly(thiol)-Verbindung. Beispiele für einsetzbare Oligo- oder Poly(thiol)-Verbindungen sind insbesondere o-, p- oder m-Dimercaptobenzol oder Ester der Thioglykol- oder 3-Mercaptopropionsäure mit linearen oder verzweigten C₂- bis C₁₈-Polyolen, besonders bevorzugt 1,2-Ethan- und 1,6 Hexandithiol, α,ω-Triethylenglycoldithiol, die Thioglycolsäure- oder 3-Mercaptopropionsäureester von Ethylen-, Propylen- und Butylenglycol und von 1,6-Hexandiol, Glycerin, Trimethylolpropan und Pentaerythrit.

Demgegenüber werden als bevorzugte Reaktionspartner des Kieselsäurekondensats des Mercaptosilans (Ib) das Norbornensilan (Ia) ein Kieselsäurekondensat des Norbornensilans (Ia) und/oder eine Oligo- oder Poly(norbornen)-Verbindung eingesetzt. Beispiele für einsetzbare Oligo- oder Poly(norbornen)-Verbindungen sind insbesondere Diels-Alder-Additionsprodukte von Cyclopentadien mit Di- oder Multi(meth)acrylaten, Ester oder Urethane von 5-Norbornen-2-methanol oder 5-Norbornen-2-ol mit Di- oder Polycarbonsäuren oder Di- oder Polyisocyanaten, besonders bevorzugt Diels-Alder-Additionsprodukte von Cyclopentadien mit mehrfunktionellen (Meth)acrylsäureestern von Ethylen-, Propylen- und Butylenglycol und von 1,6-Hexandiol, Glycerin, Trimethylolpropan und Pentaerythrit sowie die mehrfunktionellen Ester aus 5-Norbornen-2-methanol und Malon-, Malein-, Bernstein-, Glutar-, Terephthal- und Isophthalsäure.

Die Thiol-En-Polymerisation der erfindungsgemäß verwendeten Zusammensetzung führt dabei zur Addition von Thiol-Gruppen der einen Komponente an C=C-Doppelbindungen der anderen Komponente, und es bilden sich als Reaktionsprodukte anorganisch-organische Kompositmaterialien mit einer hohen mechanischen Härte und Festigkeit, die über der von herkömmlichen En-Thiol-Polymerisaten liegt. Weiter tritt bei der Thiol-En-Polymerisation überraschenderweise nur ein sehr geringer Polymerisationsschrumpf auf, was insbesondere bei der Verwendung der Zusammensetzungen als Komponente von erfindungsgemäßen dentalen Füllungsmaterialien oder dentalen Zemente von Vorteil ist.

Zur Initierung der Thiol-En-Polymerisation werden vorzugsweise thermische und/oder Photoinitiatoren der erfindungsgemäß eingesetzten Zusammensetzung zugegeben.

Bevorzugte Beispiele für thermische Initiatoren sind die bekannten Peroxide, wie Dibenzoylperoxid, Dilaurylperoxid, tert.-Butylperoctoat oder tert.-Butylperbenzoat sowie Azobisisobutyroethylester, Benzpinakol oder 2,2-Dimethylbenzpinakol.

Beispiele für geeignete Photoinitiatoren sind Benzophenon, Benzoin sowie deren Derivate oder α-Diketone und deren Derivate, wie 9,10-Phenanthrenchinon, Diacetyl oder 4,4-Dichlorbenzil. Bevorzugt werden 2,2-Methoxy-2-phenylacetophenon und Campherchinon und besonders bevorzugt α-Diketone in Kombination mit Aminen als Reduktionsmittel, wie z.B. Cyanoethylmethylanilin, Methylaminoethylmethacrylat, Triethanolamin, N,N-Dimethyl-sym.-xylidin, eingesetzt. Für die Herstellung von schmierschichtfreien erfindungsgemäßen Dentalmaterialien sind besonders geeignete Photoinitiatoren Acylphosphine, wie z.B. 2,4,6-Trimethylbenzoyldiphenyl- oder Bis(2,6-dichlorbenzoyl)-4-N-propylphenylphosphinoxid.

Als Initiatoren für eine bei Raumtemperatur durchgeführte Pclymerisation werden Radikalkettenstarter, wie z.B. Benzoyl- oder Laurylperoxid in Kombination mit Aminen, wie z.B. N,N-Dimethylsym.-xylidin oder N,N-Dimethyl-p-toluidin, verwendet.

Die erfindungsgemäß eingesetzte Zusammensetzung kann entweder als solche oder in zumindest teilweise polymerisierter Form als Dentalmaterial eingesetzt werden.

Weiter können die erfindungsgemäß eingesetzten Zusammensetzungen ebenfalls Füllstoffe enthalten. Beispiele für bevorzugte Füllstoffe sind Quarz-, Glaskeramik- und Glaspulver, insbesondere Bariumsilikatglas-Pulver, Lithium/Aluminium-Silikatglas-Pulver und Bariumglas-Pulver, Aluminium- oder Siliciumoxide, feinstteilige Kieselsäuren, insbesondere pyrogene oder gefällte Kieselsäuren, und röntgenopake Füllstoffe, wie Ytterbiumtrifluorid.

Eine besonders bevorzugte erfindungsgemäß eingesetzte Zusammensetzung enthält
(a) 5 bis 80, insbesondere 10 bis 60 Gew.%, bezogen auf die Zusammensetzung, Kieselsäurekondensat von dem Mercaptosilan (Ib) oder Norbornensilan (Ia), sowie
(b) 0 bis 50, insbesondere 0 bis 30 Gew.%, bezogen auf die Zusammensetzung, weiteren hydrolytisch kondensierbaren Verbindungen, ggf. in Form von Kondensaten,
(c) 5 bis 80, insbesondere 20 bis 70 Gew.%, bezogen auf die Zusammensetzung, Reaktionspartner für Thiol-En-Polymerisation,
(d) 0,1 bis 5, insbesondere 0,2 bis 2 Gew.%, bezogen auf die Zusammensetzung, Polymerisationsinitiator,
   und/oder
(e) 0 bis 90 Gew.-%, insbesondere 0 bis 80 Gew.-%, bezogen auf die Zusammensetzung, Füllstoffe.

Besonders bevorzugt wird die Zusammensetzung als dentaler Zement, dentales Füllungsmaterial oder dentales Bonding für Füllungsmaterialien eingesetzt. Die Verwendung der Zusammensetzung erfolgt dabei insbesondere dadurch, daß sie auf den zu behandelnden Bereich eines künstlichen oder natürlichen Zahnes aufgebracht und insbesondere durch eine Thiol-En-Polymerisation gehärtet wird.

Dabei erweist es sich als besonderer Vorteil der erfindungsgemäß eingesetzten Zusammensetzungen, daß diese in vorteilhafter Weise einerseits einen geringen Polymerisationsschrumpf zeigen und andererseits Kompositmaterialien mit hoher mechanischer Festigkeit ergeben. Eine derartige Kombination von Eigenschaften ist gerade bei Dentalmaterialien von besonderer Bedeutung.

Weiter ist es auch möglich, den erfindungsgemäß eingesetzten Zusammensetzungen für Dentalmaterialien übliche Hilfs- und Zusatzstoffe, wie z.B. Farbstoffe, Pigmente, Thixotropiehilfsmittel, Stabilisatoren, Weichmacher, Aromastoffe oder mikrobizide Wirkstoffe, zuzugeben.

Weiterer Gegenstand der Erfindung ist schließlich ein Dentalmaterial, welches sich durch Gehalt an der vorstehend beschriebenen Zusammensetzung auszeichnet.

Die Erfindung wird im folgenden anhand von Beispielen näher erläutert.

### Beispiel 1: 3-(Bicyclo[2.2.1]hept-2-en-5-methyl-5-carbonyloxy)propyltrimethoxysilan (1)

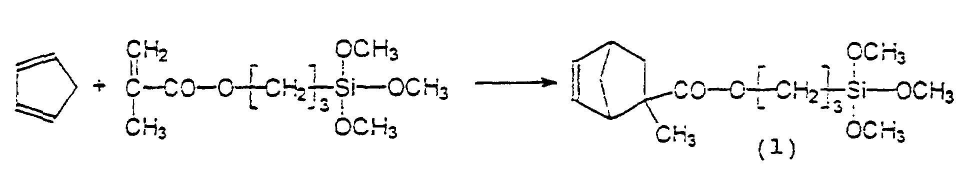

In einem Sulfierkolben, der über eine Destillationsbrücke mit einer Crackaparatur für Cyclopentadien verbunden ist, werden 59,1 g (0,20 Mol) 3-Methacryloyloxypropyltrimethoxysilan und etwas Hydrochinonmonomethylether (MEHQ) vorgelegt. Nach Spülen mit Argon leitet man Cyclopentadien in das Reaktionsgefäß ein, wobei gleichzeitig gerührt und auf 85 bis 90°C erwärmt wird. Nach 33 Stunden ist die Reaktion beendet, und gebildetes Dicyclopentadien wird bei 0,05 mbar abdestilliert. Nach anschließender fraktionierter Destillation im Hochvakuum erhält man das Norbornensilan (1) (Kp.: 98-100°C/7,0 mPa) als farblose Flüssigkeit in 70 %-iger Ausbeute.

| | | | |
|---|---|---|---|
| Elementaranalyse | C₁₅H₂₆O₅Si | Ber.: C 57,30 | H 8,33 |
| | (314,45) | Gef.: C 57,12 | H 8,31 |

- ¹H NMR (CDCl₃, 90 MHz) :: 0,38-0,83 (2H, m, Si-CH₂), 1,10-2,57 (9H, m, C-CH₃ und 3 x -CH₂), 2,72-3,17 (2H, m, >CH-), 3,57 (9H, s, O-CH₃), 4,07 (2H, t, O-CH₂) und 6,00-6,28 (2H, m, =CH-).
- IR (Film, cm⁻¹):: 1725 (C=O, 725 (H-C=C).

### Beispiel 2: 3-(Bicylco[2.2.1]hept-2-en-5-carbonyloxy)propyldimethoxymethylsilan (2)

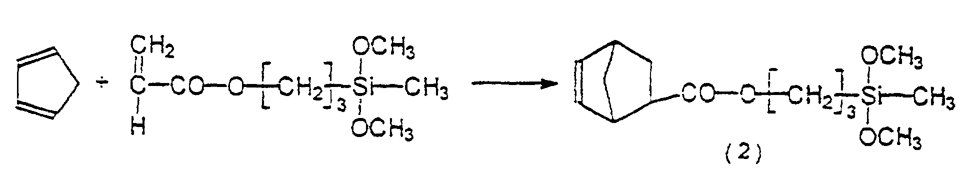

Man geht wie in Beispiel 1 beschrieben vor, setzt jedoch als Dienophil 3-Acryloyloxypropylmethyldimethoxysilan ein. Die Umsetzung ist schon nach 7 Stunden beendet. Nach Abtrennen des Dicyclopentadiens ergibt die fraktionierte Destillation im Hochvakuum das Norbornensilan (2) (Kp.: 94-96°C/4,0 mPa) als farblose Flüssigkeit in 89 %-iger Ausbeute.

| | | | |
|---|---|---|---|
| Elementaranalyse | C₁₄H₂₄O₄Si | Ber.: C 59,12 | H 8,50 |
| | (284,42) | Gef.: C 58,58 | H 8,23 |

- ¹H NMR (CDCl₃, 90 MHz):: 0,13 (3H, s, Si-CH₃), 0,55-0,77 (2H, m, Si-CH₂), 1,22-2,07 (6,5H, m, -CH₂-/>CH-COₑₓₒ), 2,83-3,30 (2,5H, m, >CH-/>CH-COₑₙ₋ _{do}), 3,55 (6H, s, O-CH₃), 4,00 (2H, t, O-CH₂) und 5,88-6,27 (2H, m, =CH-)
- IR (Film, cm⁻¹):: 1732 (C=O, 712 (H-C=C).

### Beispiel 3: 3-(Bicyclo[2.2.1]hept-2-en-5-methoxycarbamoyl)propyltriethoxysilan (3)

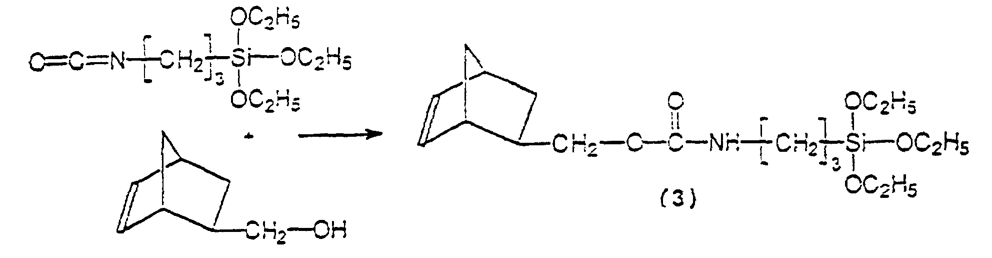

Zu einer Mischung von 24,8 g (0,20 Mol) 5-Norborn-2-en-methanol, 0,1 g Di-n-butylzinncarboxylat (Metatin 801) als Katalysator und 40 mg Hydrochinonmonomethylether (MEHQ) werden 49,2 g (0,20 Mol) 3-Isocyanatopropyltriethoxysilan (IPTES) unter Rühren und Kühlen zugetropft. Anschließend rührt man 72 Stunden lang bei Raumtemperatur, bis die Isocyanatgruppen verbraucht sind. Die fraktionierte Destillation ergibt das Silan (3) (Kp.: 136-140°C bei 5,0 mPa) als farblose Flüssigkeit in 76 %-iger Ausbeute.

| | | | |
|---|---|---|---|
| Elementaranalyse | C₁₈H₃₃O₅Si | Ber.: C 58,19 | H 8,95 |
| | (371,55) | Gef.: C 57,28 | H 9,00 |

- ¹H NMR (CDCl₃, 90 MHz):: 0,33-0,73 (2H, m, Si-CH₂), 1,12-1,92 (13H, m, 3 × -CH₂-, 3 x -CH₃), 2,23-2,37 (1H, m, >CH-CH₂-O), 2,70-2,93 (2H, m,-CH<), 3,17 (2H, q, NH-CH₂-), 3,67-3,97 (8H, m, O-CH₂-), 4,90-5,10 (1H, s, -NH-) und 5,85-6,20 (2H, m, =CH-).
- IR (Film, cm⁻¹):: 3342 (NH-Urethan), 1726 (C=O), 721 (H-C=C)

### Beispiel 4: Di(norbornen)verbindung (4)

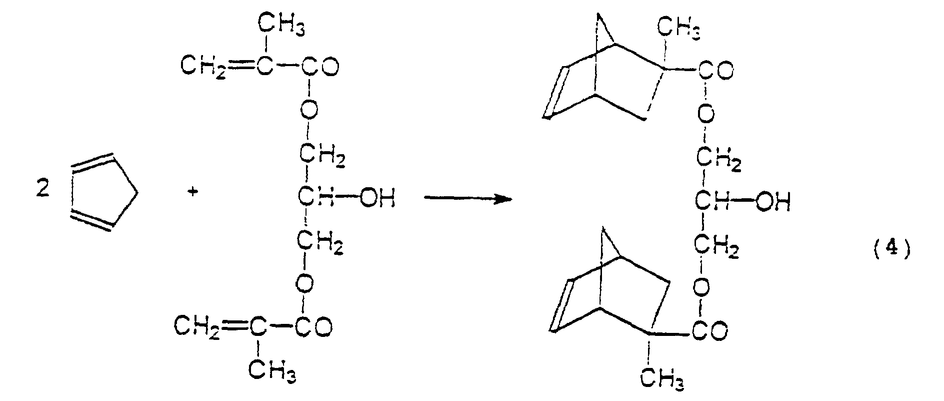

Man geht wie in Beispiel 1 beschrieben vor, setzt jedoch als Dienophil 137,0 g (0,6 Mol) käufliches Glycerindimethacrylat (Firma Röhm) ein. Nach vollständiger Umsetzung der Methacrylatgruppen zieht man die flüchtigen Bestandteile im Feinvakuum ab und erhält die gewünschte Di(norbornen)verbindung (4), welche ohne Reinigung als Reaktionspartner für eine Thiol-En-Polymerisation für weitere Umsetzungen eingesetzt werden kann.
- IR (Film, cm⁻¹):: 3496 (OH, breit), 1730 (C=O), 1572 (C=C, Norbornen), 725 (H-C=C)

### Beispiel 5: Di(norbornen)silan (5)

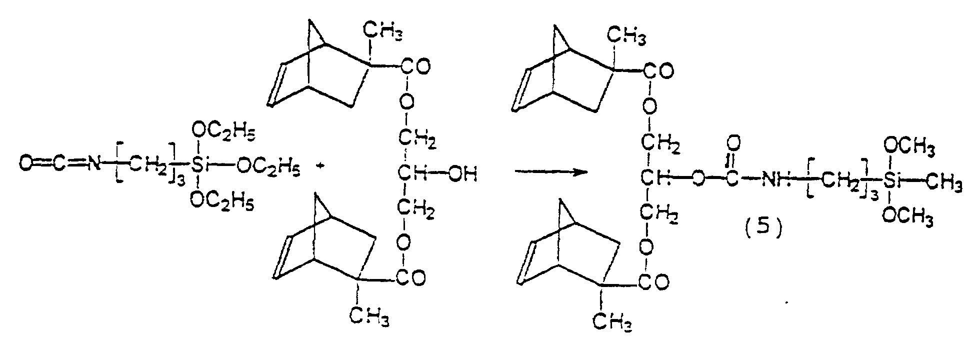

Man geht wie in Beispiel 3 beschrieben vor, setzt jedoch eine Lösung von 14,4 g (40 mMol) der OH-gruppenhaltigen Di(norbornen)verbindung (4) in ca. 20 ml absolutem THF mit 9,9 g (40 mMol) IPTES um. Man läßt 48 Stunden lang bei Raumtemperatur Rühren und engt im Feinvakuum bis zur Gewichtskonstanz ein, woraufhin das Di(norbornen)silan (5) in nahezu quantitativer Ausbeute erhalten wird.
- IR (Film, cm⁻¹):: 3380 (NH-Urethan), 1733 (C=O), 725 (H-C=C)

### Beispiel 6: Herstellung eines Kieselsäurekondensates auf der Basis des Norbornensilans (1)

Es werden 20 mMol Norbornensilan (1) und 20 mMol Dimethyldimethoxysilan in 50 ml wasserfreiem Ethanol gelöst. Nach Zugabe von 50 mMol Wasser als Mischung mit 5 ml Ethanol und einiger Tropfen 0,1 molarer ethanolischer Essigsäurelösung erwärmt man 5 Stunden lang unter Rückfluß und läßt über Nacht Rühren. Nach Entfernen flüchtiger Komponenten im Vakuum kann das gebildete harzartige Kieselsäurekondensat (7 g) für die Thiol-En-Polymerisation eingesetzt werden.

### Beisciel 7: Herstellung eines dentalen Bondings

Zu 7 g Kieselsäurekondensat gemäß Beispiel 6 werden 21 mg Campherchinon (0,3 Gew.-%) als Initiator und 35 mg (0,5 Gew.-%) 2-Ethyl-hexyl-N,N-dimethyl-4-aminobenzoat gegeben. Anschließend fügt man 5,0 mMol Pentaerythrittetra(3-mercaptopropionat) (PETMP) (Firma Evans Chemetics) als Reaktionspartner für die Thiol-En-Polymerisation zu, gießt die Mischung als Film aus und bestrahlt 60 Sekunden lang mit einer dentalen Lichtquelle, nämlich Heliomat (Firma Vivadent). Es bildet sich ein fester gut haftender Film. Von dem eingesetzten harzartigen Kieselsäurekondensat und dem Polymerisat wurden die Dichten nach der Auftriebsmethode bestimmt, wobei sich aus der Dichtedifferenz die Volumenänderung während der Thiol-En-Polymerisation ergab. Der ermittelte ΔV-Wert von nur 0,5 % war deutlich niedriger als der konventioneller Bondings auf Methacrylatbasis. So liegt der Volumenschrumpf bei der Härtung eines herkömmlichen Bondings der nachstehenden Zusammensetzung bei 7,5 %.

### Zusammensetzung herkömmliches Bonding

| **Komponente** | **Gew.-%** |
|---|---|
| Bis-GMA* | 60 |
| Triethylenglycoldimethacrylat | 39,26 |
| Cyanoethylmethylanilin | 0,5 |
| Campherchinon | 0,24 |
| Bis-GMA* = 2,2-Bis-4-(3-methacryloxy-2-hydroxy-propoxy)-phenylpropan | |

### Beispiel 8: Herstellung eines Kieselsäurekondensates aus einer Mischung des Di(norbornen)silans (5) und der Di(norbornen)verbindung (4)

40 mMol Di(norbornen)silan (5) werden mit 16 mMol Di(norbornen)verbindung (4) und 25 ml Essigsäureethylester versetzt, zur Hydrolyse/Kondensation der Triethoxysilyl-Gruppen mit der entsprechenden Menge Wasser (10 mMol H₂O pro OC₂H₅-Gruppe) gemischt und analog Beispiel 6 aufgearbeitet, wobei sich in praktisch quantitativer Ausbeute ein Harz mit einer Viskosität von 2800 Pa·s (25/40°C) bildet. Dieses Harz kann als Bestandteil eines Dentalzementes eingesetzt werden.

### Beispiel 9: Synthese der Di(norbornen)verbindung (6)

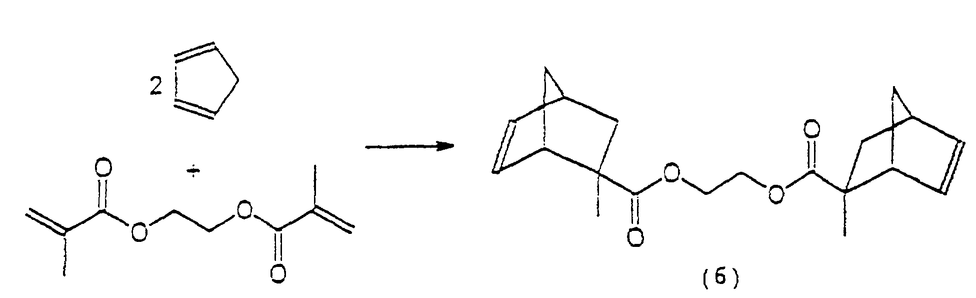

Man geht wie in Beispiel 1 beschrieben vor, setzt jedoch als Dienophil 118,9 g (0,6 Mol) käufliches Ethylenglycoldimethacrylat (Firma Fluka) ein. Nach vollständiger Umsetzung der Methacrylatgruppen zieht man die flüchtigen Bestandteile im Feinvakuum ab und erhält die gewünschte Di(norbornen)verbindung (6), welche eine Viskosität von ca. 200 mPa·s (25°C) aufweist. Dabei zeigt das HPLC-Diagramm, daß das Produkt mindestens 5 verschiedene Komponenten, wahrscheinlich Konfigurationsisomere enthält. Cyclopentadien, Dicyclopentadien oder Ethylenglycoldimethacrylat sind jedoch nicht mehr nachweisbar. Die Di(norbornen)verbindung (6) kann als Reaktivverdünner für einen dentalen Zement und als Reaktionspartner für eine Thiol-En-Polymerisation verwendet werden. So reduziert sich z.B. die Viskosität des Harzes aus Beispiel 8 durch Verdünnen mit dem gleichen Gewichtsanteil an Di(norbornen)verbindung (6) auf 8 Pa·s.

### IR-Daten von (6):

- IR (Film, cm⁻¹):: 1730 (C=O), 1572 (C=C, Norbornen), 724 (H-C=C)

### Beispiel 10: Synthese und Hydrolyse/Kondensation des Norbornensilans (7)

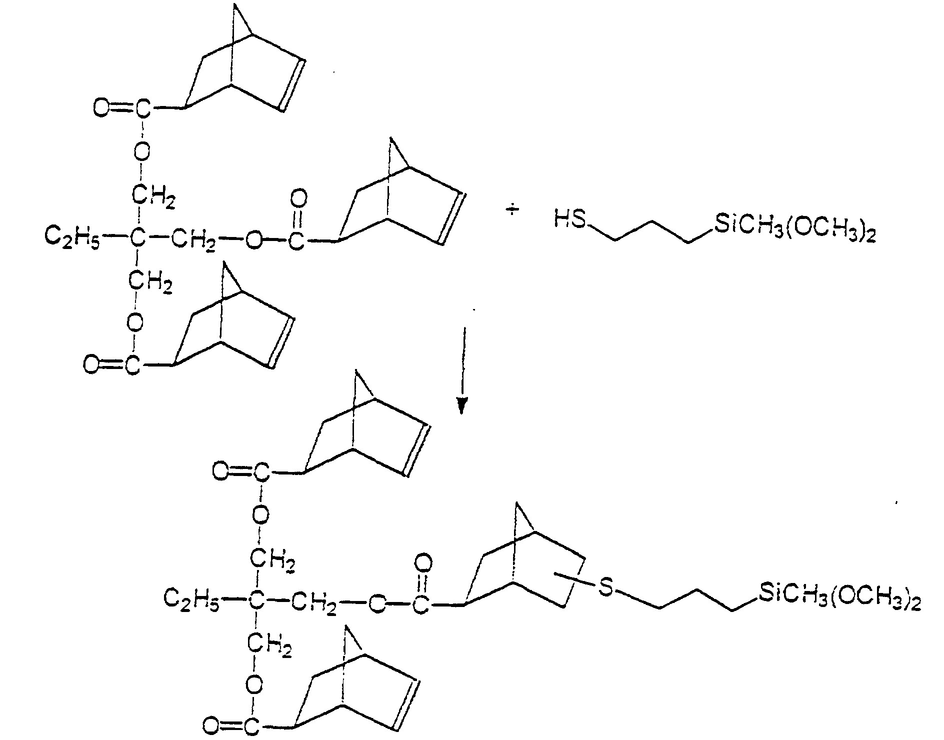

42,7 g (90 mMol) des Diels-Alder-Adduktes von 1 Mol Trimethylolpropantriacrylat mit 3 Mol Cyclopentadien werden in 90 ml Ethylacetat gelöst. Dazu tropft man unter Schutzgas 16,2 g (90 mMol) Mercaptopropylmethyldimethoxysilan zu. Nach erfolgter Thioladdition (Überprüfung mittels Iod-Test) kann die Produktlösung hydrolysiert werden. Dazu gibt man 5,8 g (18 mMol) Dinorbornenverbindung (4) als Reaktivverdünner und 2,6 g Wasser als 0,1 n HCl zur Produktlösung hinzu und rührt bei Raumtemperatur. Nach vollständiger Umsetzung wird mit Wasser ausgeschüttelt, filtriert und bei 40°C einrotiert. Nach Abzug der restlichen flüchtigen Bestandteile im Ölpumpenvakuum bei 40°C wird ein zähflüssiges Harz erhalten (ca. 820 Pa·s bei 25°C).

### Beispiel 11: Herstellung eines Dentalzementes

In einem Kapselmischer wird aus den folgenden Komponenten ein Dentalzement hergestellt:

| **Komponenten** | **Gehalt (Gew.-%)** |
|---|---|
| Harz aus Beispiel 8 | 11,7 |
| Reaktivverdünner aus Beispiel 9 | 11,7 |
| PETMP (Evans Chemetics) | 16,3 |
| Silanisierte pyrogene Kieselsäure Ox-50 (Firma Degussa) | 41,3 |
| Ytterbiumfluorid (Firma Rhone-Poulenc) | 18,7 |
| Campherchinon | 0,2 |
| Eusolex 6007 (2-Ethylhexyl-dimethylaminobenzoat) (Firma Merck) als Aktivator | 0,1 |

Mit dem Zement werden Prüfkörper hergestellt, die anschließend mit einer dentalen Lichtquelle, Spectramat (Firma Vivadent), 10 Minuten lang belichtet werden. Danach wurden gemäß ISO 4049 die Biegefestigkeit zu 89 MPa und der E-Modul zu 5,5 GPa bei 24 Stunden lang gelagerten Proben bestimmt. Der Polymerisationsschrumpf betrug lediglich 0,9 Vol.-%.

## Patentansprüche

1. Verwendung einer Zusammensetzung als Dentalmaterial, **dadurch gekennzeichnet, daß** die Zusammensetzung einen Gehalt an
(a) mindestens einem Kieselsäurekondensat eines hydrolysierbaren und polymerisierbaren Norbornensilans der allgemeinen Formel (Ia) 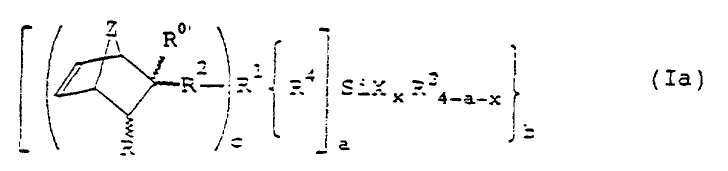 wobei die Variablen R⁰, R, R¹, R², R³, R⁴, R⁵, R⁶, X, Y, Z, a, b, c, x , sofern nicht anders angegeben, unabhängig voneinander die folgenden Bedeutungen haben:
R⁰ = C₁- bis C₈-Alkyl oder H;
R = C₁- bis C₈-Alkyl oder -Alkenyl oder C₆- bis C₁₀-Aryl, wobei diese Reste durch O- oder S-Atome oder durch -O-CO-, -CO-O- oder NH-Gruppen unterbrochen sein können oder diese Atome oder Gruppen endständig tragen können, oder H oder R²-R¹-R⁴-SiXₓR³₃₋ₓ;
R¹ = entfällt oder C₁- bis C₈-Alkylen oder C₆- bis C₁₄-Arylen, -Arylenalkylen oder -Alkylenarylen, wobei diese Reste durch O- oder S-Atome oder durch -O-CO-, -CO-O- oder NH-Gruppen unterbrochen sein können oder diese Atome oder Gruppen endständig tragen können;
R² = entfällt oder C₁- bis C₈-Alkylen oder C₆- bis C₁₄-Arylen, -Arylenalkylen oder -Alkylenarylen, wobei diese Reste durch O- oder S-Atome oder durch -O-CO-, -CO-O- oder NH-Gruppen unterbrochen sein können oder diese Atome oder Gruppen endständig tragen können;
R³ = C₁- bis C₁₀-Alkyl oder -Alkenyl oder C₆- bis C₁₀-Aryl, wobei diese Reste durch O- oder S-Atome unterbrochen sein können oder diese Atome endständig tragen können;
R⁴ = -(CHR⁶-CHR⁶)ₙ-, mit n = 0 oder 1, -CHR⁶-CHR⁶-S-R⁵-, -CO-S-R⁵-, -CHR⁶-CHR⁶-NR⁶-R⁵-, -S-R⁵, -Y-CO-NH-R⁵- oder -CO-O-R⁵-;
R⁵ = C₁- bis C₈-Alkylen oder C₆- bis C₁₀-Arylen, wobei diese Reste durch O- oder S-Atome oder durch -O-CO-, -CO-O- oder NH-Gruppen unterbrochen sein können oder diese Atome oder Gruppen endständig tragen können;
R⁶ = H, C₁- bis C₁₀-Alkyl oder C₆- bis C₁₀-Aryl;
X = eine hydrolysierbare Gruppe, nämlich Halogen, OH oder Alkoxy;
Y = O, S oder NR⁶;
Z = O oder CHR⁶ ;
a = 1, 2 oder 3;
b = 1, 2 oder 3;
c = 1 bis 6; und
x = 1, 2 oder 3;
und mit der Maßgabe, daß
a und/oder b = 1
und
a+x = 2, 3 oder 4.
oder
(b) mindestens einem Kieselsäurekondensat eines hydrolysierbaren und polymerisierbaren Mercaptosilans der allgemeinen Formel (Ib)
aufweist,
[(HS-R⁷)_{f}R⁸]_{g}SiXₕR⁹ _{4-g-h} (Ib) (Ib)
wobei die Variablen R⁷, R⁸, R⁹, X, f, g und h, sofern nicht anders angegeben, unabhängig voneinander die folgenden Bedeutungen haben:
R⁷ = C₁- bis C₁₀-Alkylen oder -Alkenylen oder C₆-bis C₁₄-Arylen oder -Alkylarylen, wobei diese Reste durch O- oder S-Atome oder -CO-O- oder -O-CO-Gruppen unterbrochen sein können oder diese Atome oder Gruppen endständig tragen können;
R⁸ = C₁- bis C₁₀-Alkylen oder -Alkenylen oder C₆-bis C₁₄-Arylen oder -Alkylarylen, wobei diese Reste durch O- oder S-Atome oder -CO-O- oder -O-CO-Gruppen unterbrochen sein können oder diese Atome oder Gruppen endständig tragen können;
R⁹ = C₁- bis C₁₀-Alkyl oder -Alkenyl oder C₆- bis C₁₄-Aryl- oder -Alkylaryl, wobei diese Reste durch O- oder S-Atome oder -CO-O- oder -O-CO-Gruppen unterbrochen sein können oder diese Atome oder Gruppen endständig tragen können;
X = eine hydrolysierbare Gruppe, nämlich Halogen, Hydroxy oder Alkoxy;
g = 1, 2 oder 3;
f = 1, 2, 3 oder 4; und
h = 1, 2 oder 3.

2. Verwendung nach Anspruch 1, **dadurch gekennzeichnet, daß** mindestens eine der Variablen der Formel (Ia), sofern nicht anders angegeben, unabhängig von den übrigen Variablen, die folgende Bedeutung hat:
R⁰ = H oder C₁- bis C₅-Alkyl;
R = H oder C₁- bis C₅-Alkyl;
R¹ = C₁- bis C₈-Alkylen, wobei diese Reste durch O- oder S-Atome oder durch -CO-O- oder -O-CO-Gruppen unterbrochen sein können oder diese Atome oder Gruppen endständig tragen können;
R² = C₁- bis C₈-Alkylen, wobei diese Reste durch O- oder S-Atome unterbrochen sein können oder diese Atome endständig tragen können;
R³ = CH₃, C₂H₅ oder Phenyl;
R⁴ = (CHR⁶-CHR⁶)ₙ-, -S-R⁵, Y-CO-NH-R⁵- oder -CO-O-R⁵-;
R⁵ = C₁- bis C₈-Alkylen, wobei diese Reste durch O- oder S-Atome oder durch -CO-O- oder -O-CO-Gruppen unterbrochen sein können oder diese Atome oder Gruppen endständig tragen können;
R⁶ = H, C₁- bis C₅-Alkyl;
X = OCH₃ oder OC₂H₅;
Y = O oder S;
Z = CH₂;
a = 1;
b = 1;
c = 1 oder 2;
x = 2 oder 3;
und/oder
a+x = 3 oder 4.
und
mindestens eine der Variablen der Formel (Ib), sofern nicht anders angegeben, unabhängig von den übrigen Variablen, die folgende Bedeutung hat:
R⁷ = C₁- bis C₁₀-Alkylen oder C₆- bis C₁₄-Arylen, wobei diese Reste durch O- oder S-Atome oder -CO-O- oder -O-CO-Gruppen unterbrochen sein können oder diese Atome oder Gruppen endständig tragen können;
R⁸ = C₁- bis C₁₀-Alkylen oder C₆- bis C₁₄-Arylen, wobei diese Reste durch O- oder S-Atome oder -CO-O- oder -O-CO-Gruppen unterbrochen sein können oder diese Atome oder Gruppen endständig tragen können;
X = OCH₃, OC₂H₅ oder Cl;
R⁹ = C₁- bis C₁₀- Alkyl;
und/oder
h = 2 oder 3.

3. Verwendung nach Anspruch 1 oder 2, **dadurch gekennzeichnet, daß**
a = 1 und b = 1
und damit das Norbornensilan der allgemeinen Formel (IIa) entspricht 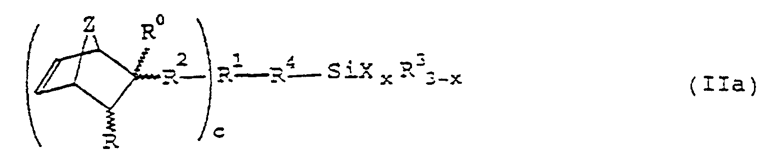 und daß
g = 1
und damit das Mercaptosilan der allgemeinen Formel (IIb) entspricht:
(HS-R⁷)_{f}R⁸-SiXₕR⁹ ₃₋ₕ (IIb)

4. Verwendung nach Anspruch 1 oder 2, **dadurch gekennzeichnet, daß**
a = 1 und c = 1
und damit das Norbornensilan der allgemeinen Formel (IIIa) entspricht: 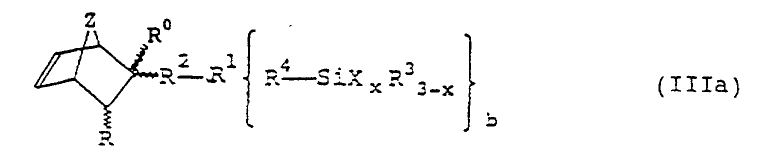 und daß
f = 1 und g = 1
und damit das Mercaptosilan der allgemeinen Formel (IIIb) entspricht:
HS-R⁷-R⁸-SiXₕR⁹ ₃₋ₕ (IIIb)

5. Verwendung nach Anspruch 1 oder 2, **dadurch gekennzeichnet, daß**
b = 1 und c = 1
und damit das Norbornensilan der allgemeinen Formel (IVa) entspricht: 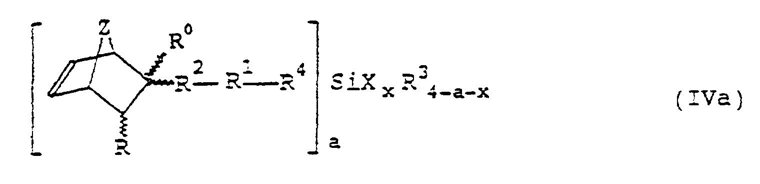 und daß
f = 1
und damit das Mercaptosilan der allgemeinen Fromel (IVb) entspricht:
[HS-R⁷-R⁸]_{g}SiXₕR⁹ _{4-g-h} (IVb)

6. Verwendung nach Anspruch 1 oder 2, **dadurch gekennzeichnet, daß**
a = 1 und b = 1 und c = 1
und damit das Norbornensilan der allgemeinen Formel (Va) entspricht.

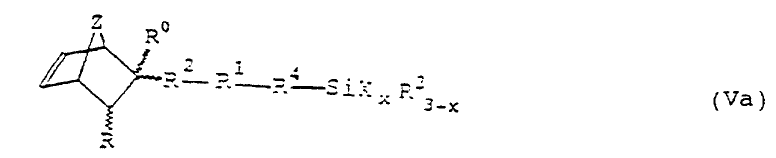

7. Verwendung nach einem der Ansprüche 1 bis 6, **dadurch gekennzeichnet, daß**
die Zusammensetzung weitere hydrolytisch kondensierbare Verbindungen enthält.

8. Verwendung nach Anspruch 7, **dadurch gekennzeichnet, daß** als weitere hydrolytisch kondensierbare Verbindungen mindestens ein Silan der allgemeinen Formel (VIII) eingesetzt wird
R¹⁰ ₖ(Z'R¹¹)ₘSiX'₄₋₍ₖ₊ₘ₎ (VIII)
wobei R¹⁰, Z',R¹¹, X', k und m, sofern nicht anders angegeben, unabhängig voneinander die folgenden Bedeutungen haben:
R¹⁰ = C₁- bis C₈-Alkyl, C₁- bis C₁₂-Alkenyl oder C₆- bis C₁₄-Aryl,
R¹¹ = C₁- bis C₈-Alkylen, C₁- bis C₁₂-Alkenylen oder C₆-bis C₁₄-Arylen,
X' H, Halogen, OH, C₁- bis C₈-Alkoxy,
Z' = Mercapto-, Carboxy-, Acryl, Methacryl-, Allyl-, Vinyl- oder Vinylether-Gruppe;
k = 0, 1, 2 oder 3,
m = 0, 1, 2 oder 3, und
k+m = 1, 2 oder 3.

9. Verwendung nach Anspruch 7 oder 8, **dadurch gekennzeichnet, daß** als weitere hydrolytisch kondensierbare Verbindungen mindestens eine Aluminium-, Titan- oder Zirkonium-Verbindung der Formel
AlR¹² ₃ oder M X"_{y} R¹³ _{z}
eingesetzt wird, wobei M, R¹², R¹³, X", y und z unabhängig voneinander die folgenden Bedeutungen haben:
M = Ti oder Zr,
R¹² = Halogen, OH, C₁- bis C₈-Alkoxy,
R¹³ = R, wie in Anspruch 1 definiert,
X" = Halogen, OH oder C₁- bis C₈-Alkoxy,
y = 1 bis 4, insbesondere 2 bis 4, und
z = 0 bis 3, insbesondere 0 bis 2.

10. Verwendung nach einem der Ansprüche 1 bis 9, **dadurch gekennzeichnet, daß** die Zusammensetzung das Kieselsäurekondensat des Norbornensilans oder das Kieselsäurekondensat des Mercaptosilans jeweils in Kombination mit mindestens einem Reaktionspartner für eine Thiol-En-Polymerisation enthält.

11. Verwendung nach Anspruch 10, **dadurch gekennzeichnet, daß**
(a) das Mercaptosilan, ein Kieselsäurekondensat des Mercaptosilans und/oder eine Oligo- oder Poly(thiol)-Verbindung als Reaktionspartner des Kieselsäurekondensats des Norbornensilans eingesetzt wird, und
(b) das Norbornensilan, ein Kieselsäurekondensat des Norbonensilans und/oder eine Oligo- oder Poly(norbornen)-Verbindung als Reaktionspartner des Kieselsäurekondensats des Mercaptosilans eingesetzt wird.

12. Verwendung nach Anspruch 11, **dadurch gekennzeichnet, daß**
(a) als Oligo- oder Poly(thiol)-Verbindung o-, p- oder m-Dimercaptobenzol oder Ester der Thioglykol- oder 3-Mercaptopropionsäure mit linearen oder verzweigten C₂-bis C₁₈-Polyolen, und
(b) als Oligo- oder Poly(norbornen)-Verbindung Diels-Alder-Additionsprodukte von Cyclopentadien mit Di- oder Multi(meth)acrylaten, Ester oder Urethane von 5-Norbornen-2-methanol oder 5-Norbornen-2-ol mit Di- oder Polycarbonsäuren oder Di- oder Polyisocyanaten.
eingesetzt werden.

13. Verwendung nach einem der Ansprüche 1 bis 12, **dadurch gekennzeichnet, daß** die Zusammensetzung einen Polymerisationsinitiator und insbesondere einen Initiator für die Thiol-En-Polymerisation enthält.

14. Verwendung nach einem der Ansprüche 1 bis 13, **dadurch gekennzeichnet, daß** die Zusammensetzung in zumindest teilweise polymerisierter Form eingesetzt wird.

15. Verwendung nach einem der Ansprüche 1 bis 14, **dadurch gekennzeichnet, daß** die Zusammensetzung
(a) 5 bis 80, insbesondere 10 bis 60 Gew.%, bezogen auf die Zusammensetzung, Kieselsäurekondensat des Mercaptosilans oder Kieselsäurekondensat des Norbornensilans, sowie
(b) 0 bis 50, insbesondere 0 bis 30 Gew.%, bezogen auf die Zusammensetzung, weiteren hydrolytisch kondensierbaren Verbindungen, ggf. in Form von Kieselsäurekondensaten davon,
(c) 5 bis 80, insbesondere 20 bis 70 Gew.%, bezogen auf die Zusammensetzung, Reaktionspartner für eine Thiol-En-Polymerisation,
(d) 0,1 bis 5, insbesondere 0,2 bis 2 Gew.%, bezogen auf die Zusammensetzung, Polymerisationsinitiator,
und/oder
(e) 0 bis 90 Gew.-%, insbesondere 0 bis 80 Gew.-%, bezogen auf die Zusammensetzung, Füllstoffe
enthält.

16. Verwendung nach einem der Ansprüche 1 bis 15, **dadurch gekennzeichnet, daß** die Zusammensetzung als dentaler Zement, dentales Füllungsmaterial oder Bonding für dentales Füllungsmaterial eingesetzt wird.

17. Verwendung nach einem der Ansprüche 1 bis 16, **dadurch gekennzeichnet, daß** die Zusammensetzung auf den zu behandelnden Bereich eines künstlichen oder natürlichen Zahnes aufgebracht und einer Thiol-En-Polymerisation unterworfen wird.

18. Dentalmaterial, **dadurch gekennzeichnet, daß** es die in einem der Ansprüche 1 bis 15 definierte Zusammensetzung enthält.

## Claims

1. Use of a composition as dental material, **characterised in that** the composition contains
(a) at least one silicic acid condensate of a hydrolysable and polymerisable norbornene silane of general formula (Ia) 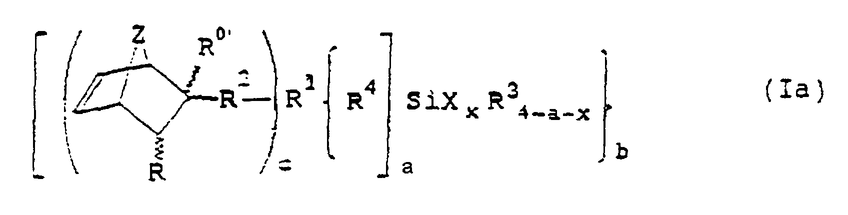 wherein the variables R⁰, R, R¹, R², R³, R⁴, R⁵, R⁶, X, Y, Z, a, b, c, x have, unless stated otherwise, independently of one another, the following meanings:
R⁰ = C₁ to C₈ alkyl or H;
R = C₁ to C₈ alkyl or alkenyl or C₆ to C₁₀ aryl, where these radicals can be interrupted by O or S atoms or by -O-CO-, -CO-O- or NH groups or can contain these atoms or groups in terminal position, or H or R²-R¹-R⁴-SiXₓR³₃₋ₓ;
r¹ = missing or C₁ to C₈ alkylene or C₆ to C₁₄ arylene, arylenealkylene or alkylenearylene, where these radicals can be interrupted by O or S atoms or by -O-CO-, -CO-O- or NH groups or can contain these atoms or groups in terminal position;
R² = missing or C₁ to C₈ alkylene or C₆ to C₁₄ arylene, arylenealkylene or alkylenearylene, where these radicals can be interrupted by O or S atoms or by -O-CO-, -CO-O- or NH groups or can contain these atoms or groups in terminal position;
R³ = C₁ to C₁₀ alkyl or alkenyl or C₆ to C₁₀ aryl, where these radicals can be interrupted by O or S atoms or can contain these atoms in terminal position;
R⁴ = -(CHR⁶-CHR⁶)ₙ-, with n = 0 or 1, -CHR⁶-CHR⁶-S-R⁵-, -CO-S-R⁵-, -CHR⁶-CHR⁶-NR⁶-R⁵-, -S-R⁵, -Y-CO-NH-R⁵- or -CO-O-R⁵-;
R⁵ = C₁ to C₈ alkylene or C₆ to C₁₀ arylene, where these radicals can be interrupted by O or S atoms or by -O-CO-, -CO-O- or NH groups or can contain these atoms or groups in terminal position;
R⁶ = H, C₁ to C₁₀ alkyl or C₆ to C₁₀ aryl;
X = a hydrolysable group, in particular halogen, OH or alkoxy;
Y = O, S or NR⁶;
Z = O or CHR⁶;
a = 1, 2 or 3;
b = 1, 2 or 3;
c = 1 to 6; and
x = 1, 2 or 3;
and with the proviso that
a and/or b = 1
and
a+x = 2, 3 or 4,
or
(b) at least one silicic acid condensate of a hydrolysable and polymerisable mercaptosilane of general formula (Ib)
[(HS-R⁷)_{f}R³]_{g}SiXₕR⁹ _{4-g-h} (Ib)
wherein the variables R⁷, R⁸, R⁹, X, f, g and h have, unless stated otherwise, independently of one another, the following meanings:
R⁷ = C₁ to C₁₀ alkylene or alkenylene or C₆ to C₁₄ arylene or alkylarylene, where these radicals can be interrupted by O or S atoms or -CO-O- or -O-CO- groups or can contain these atoms or groups in terminal position;
R⁸ = C₁ to C₁₀ alkylene or alkenylene or C₆ to C₁₄ arylene or alkylarylene, where these radicals can be interrupted by O or S atoms or -CO-O- or -O-CO- groups or can contain these atoms or groups in terminal position;
R⁹ = C₁-C₁₀ alkyl or alkenyl or C₆-C₁₄ aryl or alkylaryl, where these radicals can be interrupted by O or S atoms or -CO-O- or -O-CO- groups or can contain these atoms or groups in terminal position;
X = a hydrolysable group, in particular halogen, hydroxy or alkoxy;
g = 1, 2 or 3;
f = 1, 2, 3 or 4; and
h = 1, 2 or 3.

2. Use according to Claim 1, **characterised in that** at least one of the variables of formula (Ia) has, unless stated otherwise, independently of the other variables, the following meaning:
R⁰ = H or C₁ to C₅ alkyl;
R = H or C₁ to C₅ alkyl;
R¹ = C₁ to C₈ alkylene, where these radicals can be interrupted by O or S atoms or by -O-CO- or -O-CO- groups or can contain these atoms or groups in terminal position;
R² = C₁ to C₈ alkylene, where these radicals can be interrupted by O or S atoms or can contain these atoms in terminal position;
R³ = CH₃, C₂H₅ or phenyl;
R⁴ = (CHR⁶-CHR⁶)ₙ-, -S-R⁵, Y-CO-NH-R⁵- or -CO-O-R⁵-;
R⁵ = C₁ to C₈ alkylene, where these radicals can be interrupted by O or S atoms or by -O-CO- or -O-CO- groups or can contain these atoms or groups in terminal position;
R⁶ = H or C₁ to C₅ alkyl;
X = OCH₃ or OC₂H₅;
Y = O or S;
Z = CH2;
a = 1;
b = 1;
c = 1 or 2;
x = 2 or 3;
and/or
a+x = 3 or 4,
and
at least one of the variables of formula (Ib) has, unless stated otherwise, independently of the other variables, the following meaning:
R⁷ = C₁ to C₁₀ alkylene or C₆ to C₁₄ arylene, where these radicals can be interrupted by O or S atoms or -CO-O- or -O-CO-groups or can contain these atoms or groups in terminal position;
R⁸ = C₁ to C₁₀ alkylene or C₆ to C₁₄ arylene, where these radicals can be interrupted by O or S atoms or -CO-O- or -O-CO-groups or can contain these atoms or groups in terminal position;
X = OCH₃, OC₂H₅ or Cl;
R⁹ = C₁-C₁₀ alkyl;
and/or
h = 2 or 3.

3. Use according to Claim 1 or 2, **characterised in that**
a = 1 and b = 1
so that the norbornene silane corresponds to the general formula (IIa) 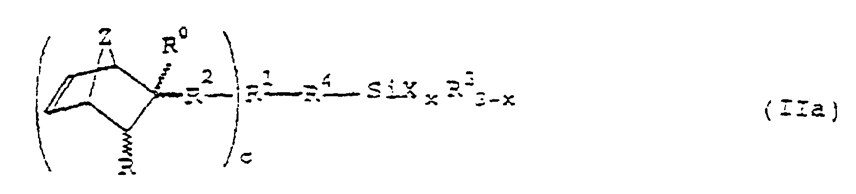 and **in that**
g = 1
so that the mercaptosilane corresponds to the general formula (IIb):
(HS-R⁷)_{f}-R⁸-SiXₕR⁹ ₃₋ₕ (IIb)

4. Use according to Claim 1 or 2, **characterised in that**
a = 1 and c = 1
so that the norbornene silane corresponds to the general formula (IIIa) : 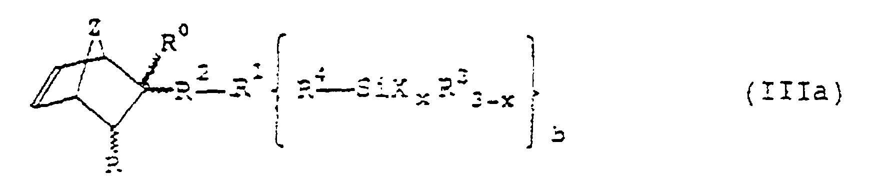 and **in that**
f = 1 and g = 1
so that the mercaptosilane corresponds to the general formula (IIIb):
HS-R⁷-R⁸-SiXₕR⁹ ₃₋ₕ (IIIb)

5. Use according to Claim 1 or 2, **characterised in that**
b = 1 and c = 1
so that the norbornene silane corresponds to the general formula (IVa): 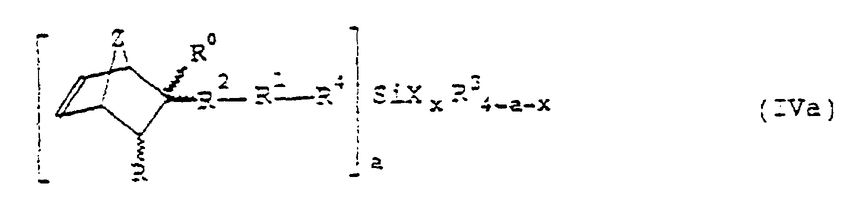 and **in that**
f = 1
so that the mercaptosilane corresponds to the general formula (IVb):
[HS-R⁷-R⁸]_{g}SiXₕR⁹ _{4-g-h} (IVb)

6. Use according to Claim 1 or 2, **characterised in that**
a = 1 and b = 1 and c = 1
so that the norbornene silane corresponds to the general formula (Va):

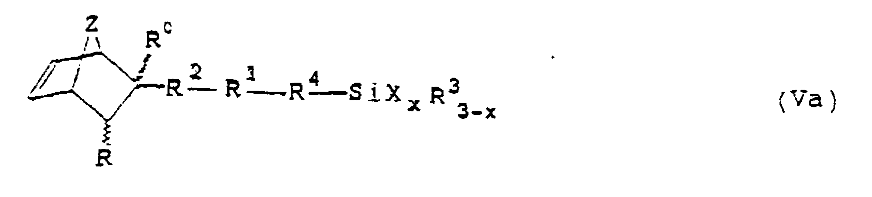

7. Use according to any one of Claims 1 to 6,
**characterised in that** the composition comprises further hydrolytically condensable compounds.

8. Use according to Claim 7, **characterised in that** the further hydrolytically condensable compounds employed comprise at least one silane of the general formula (VIII)
R¹⁰ ₖ(Z'R¹¹)ₘSiX'₄₋₍ₖ₊ₘ₎ (VIII)
wherein R¹⁰, Z', R¹¹, X', k and m have, unless stated otherwise, independently of one another, the following meanings:
R¹⁰ = C₁ to C₈ alkyl, C₁ to C₁₂ alkenyl or C₆ to C₁₄ aryl,
R¹¹ = C₁ to C₈ alkylene, C₁ to C₁₂ alkenylene or C₆ to C₁₄ arylene,
X' = H, halogen, OH, C₁ to C₈ alkoxy,
Z' = a mercapto, carboxyl, acryloyl, methacryloyl, allyl, vinyl or vinyl ether group;
k = 0, 1, 2 or 3,
m = 0, 1, 2 or 3, and
k+m = 1, 2 or 3.

9. Use according to Claim 7 or 8, **characterised in that** the further hydrolytically condensable compounds employed comprise at least one aluminium, titanium or zirconium compound of formula
AlR¹² ₃ or M X"_{y} R¹³ _{z}
wherein M, R¹², R¹³, X", y and z have, independently of one another, the following meanings:
M = Ti or Zr,
R¹² = halogen, OH, C₁ to C₈ alkoxy,
R¹³ = R, as defined in Claim 1,
X" = halogen, OH or C₁ to C₈ alkoxy,
y = 1 to 4, in particular 2 to 4, and
z = 0 to 3, in particular 0 to 2.

10. Use according to any one of Claims 1 to 9, **characterised in that** the composition contains the silicic acid condensate of the norbornene silane or the silicic acid condensate of the mercaptosilane in each case in combination with at least one reaction partner for a thiol-ene polymerization.

11. Use according to Claim 10, **characterised in that**
(a) the mercaptosilane, a silicic acid condensate of the mercaptosilane and/or an oligo- or polythiol compound is used as reaction partner of the silicic acid condensate of the norbornene silane and
(b) the norbornene silane, a silicic acid condensate of the norbornene silane and/or an oligo- or polynorbornene compound is used as reaction partner of the silicic acid condensate of the mercaptosilane.

12. Use according to Claim 11, **characterised in that**
(a) the oligo- or polythiol compound used is o-, p- or m-dimercaptobenzene or ester of thioglycolic or 3-mercaptopropionic acid with linear or branched C₂- to C₁₈-polyols and
(b) the oligo- or polynorbornene compound used is Diels-Alder addition products of cyclopentadiene with di- or multimethacrylates, esters or urethanes of 5-norbornene-2-methanol or 5-norbornen-2-ol with di- or polycarboxylic acids or di- or polyisocyanates.

13. Use according to any one of Claims 1 to 12, **characterised in that** the composition contains a polymerization initiator and especially an initiator for thiol-ene polymerization.

14. Use according to any one of Claims 1 to 13, **characterised in that** the composition is employed in at least partially polymerised form.

15. Use according to any one of Claims 1 to 14, **characterised in that** the composition comprises
(a) 5 to 80, in particular 10 to 60 wt.%, based on the composition, of silicic acid condensate of the mercaptosilane or silicic acid condensate of the norbornene silane, and
(b) 0 to 50, in particular 0 to 30 wt.%, based on the composition, of further hydrolytically condensable compounds, optionally in the form of their silicic acid condensates,
(c) 5 to 80, in particular 20 to 70 wt.%, based on the composition, of reactants for a thiol-ene polymerisation,
(d) 0.1 to 5, in particular 0.2 to 2 wt.%, based on the composition, of polymerization initiator,
and/or
(e) 0 to 90 wt.%, in particular 0 to 80 wt.%, based on the composition, of fillers.

16. Use according to any one of Claims 1 to 15 **characterised in that** the composition is employed as dental cement, dental filling material or bonding for dental filling material.

17. Use according to any one of Claims 1 to 16, **characterised in that** the composition is applied to the region to be treated of an artificial or natural tooth and is subjected to a thiol-ene polymerization.

18. Dental material, charaterised in that it comprises the composition defined in any one of Claims 1 to 15.

## Revendications

1. Utilisation d'une composition en tant que matériau dentaire, **caractérisée en ce que** la composition présente une teneur en
(a) au moins un produit de condensation de type acide silicique d'un norbornène-silane hydrolysable et polymérisable de formule générale (Ia) 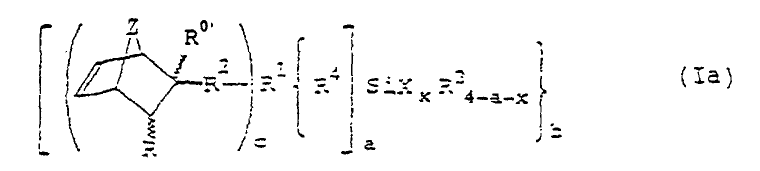 les variables R⁰, R, R¹, R², R³, R⁴, R⁵, R⁶, X, Y, Z, a, b, c, x ayant, à moins d'indication contraire, indépendamment les unes des autres, les significations suivantes :
R⁰= un groupe alkyle en C₁-C₈ ou H ;
R = un groupe alkyle ou alcényle en C₁-C₈ ou aryle en C₆-C₁₀, ces radicaux pouvant être interrompus par des atomes d'oxygène ou de soufre ou par des groupes -O-CO-, -CO-O- ou NH, ou pouvant porter ces atomes ou groupes en bout de chaîne, ou H ou un groupe R²-R¹-R⁴-SiXₓR³₃₋ₓ ;
R¹ = est absent ou représente un groupe alkylène en C₁-C₈ ou arylène, arylène-alkylène ou alkylène-arylène en C₆-C₁₄, ces radicaux pouvant être interrompus par des atomes d'oxygène ou de soufre ou par des groupes -O-CO-, -CO-O- ou NH, ou pouvant porter ces atomes ou groupes en bout de chaîne ;
R²= est absent ou représente un groupe alkylène en C₁-C₈ ou arylène, arylène-alkylène ou alkylène-arylène en C₆-C₁₄, ces radicaux pouvant être interrompus par des atomes d'oxygène ou de soufre ou par des groupes -O-CO-, -CO-O- ou NH, ou pouvant porter ces atomes ou groupes en bout de chaîne ;
R³= un groupe alkyle ou alcényle en C₁-C₁₀ ou aryle en C₆-C₁₀, ces radicaux pouvant être interrompus par des atomes d'oxygène ou de soufre ou pouvant porter ces atomes en bout de chaîne ;
R⁴= -(CHR⁶-CHR⁶)ₙ-, avec n = 0 ou 1, -CHR⁶-CHR⁶-S-R⁵-, -CO-S-R⁵-, -CHR⁶-CHR⁶-NR⁶-R⁵-, -S-R⁵, -Y-CO-NH-R⁵- ou -CO-O-R⁵- ;
R⁵= un groupe alkylène en C₁-C₈ ou arylène en C₆-C₁₀, ces radicaux pouvant être interrompus par des atomes d'oxygène ou de soufre ou par des groupes -O-CO-, -CO-O- ou NH ou pouvant porter ces atomes ou groupes en bout de chaîne ;
R⁶= H, un groupe alkyle en C₁-C₁₀ ou aryle en C₆-C₁₀;
X = un groupe hydrolysable, à savoir, un atome d'halogène ou un groupe OH ou alcoxy ;
Y = O, S ou NR⁶ ;
Z = O ou CHR⁶;
a = 1, 2 ou 3 ;
b = 1, 2 ou 3 ;
c = 1-6 ; et
x = 1, 2 ou 3;
étant entendu que
a et/ou b = 1
et
a+x = 2, 3 ou 4;
ou
(b) au moins un produit de condensation de type acide silicique d'un mercaptosilane hydrolysable et polymérisable de formule générale (Ib)
[(HS-R⁷)_{f}R⁸]_{g}SiXₜR⁹ _{4-g-h} (Ib)
les variables R⁷, R⁸, R⁹, X, f, g et h ayant, à moins d'indication contraire, indépendamment les unes des autres, les significations suivantes :
R⁷ = un groupe alkylène ou alcénylène en C₁-C₁₀ ou arylène ou alkylarylène en C₆-C₁₄, ces radicaux pouvant être interrompus par des atomes d'oxygène ou de soufre ou par des groupes ou -CO-O- ou -O-CO-, ou pouvant porter ces atomes ou groupes en bout de chaîne ;
R⁸= un groupe alkylène ou alcénylène en C₁-C₁₀ ou arylène ou alkylarylène en C₆-C₁₄, ces radicaux pouvant être interrompus par des atomes d'oxygène ou de soufre ou par des groupes -CO-O- ou -O-CO-, ou pouvant porter ces atomes ou groupes en bout de chaîne ;
R⁹ = un groupe alkyle ou alcényle en C₁-C₁₀ ou aryle ou alkylaryle en C₆-C₁₄, ces radicaux pouvant être interrompus par des atomes d'oxygène ou de soufre ou par des groupes -CO-O- ou -O-CO-, ou pouvant porter ces atomes ou groupes en bout de chaîne ;
X = un groupe hydrolysable, à savoir, un atome d'halogène ou un groupe hydroxy ou alcoxy ;
g = 1, 2 ou 3 ;
f = 1, 2, 3 ou 4 ; et
h = 1, 2 ou 3.

2. Utilisation selon la revendication 1, **caractérisée en ce qu'**au moins l'une des variables de la formule (Ia), à moins d'indication contraire, indépendamment des autres variables, a la signification suivante :
R⁰= H ou un groupe alkyle en C₁-C₅ ;
R = H ou un groupe alkyle en C₁-C₅ ;
R¹= un groupe alkylène en C₁-C₈, ce radical pouvant être interrompu par des atomes d'oxygène ou de soufre ou par des groupes -CO-O- ou -O-CO-, ou pouvant porter ces atomes ou groupes en bout de chaîne ;
R²= un groupe alkylène en C₁-C₈, ce radical pouvant être interrompu par des atomes d'oxygène ou de soufre ou pouvant porter ces atomes en bout de chaîne ;
R³= CH₃, C₂H₅ ou le groupe phényle ;
R⁴= -(CHR⁶-CHR⁶)ₙ-, -S-R⁵, -Y-CO-NH-R⁵- ou -CO-O-R⁵- ;
R⁵= un groupe alkylène en C₁-C₈, ce radical pouvant être interrompu par des atomes d'oxygène ou de soufre ou par des groupes -CO-O- ou -O-CO- ou pouvant porter ces atomes ou groupes en bout de chaîne ;
R⁶= H, un groupe alkyle en C₁-C₅;
X = OCH₃ ou OC₂H₅ ;
Y = O ou S ;
Z = CH₂;
a = 1;
b = 1;
c = 1 ou 2;
x = 2 ou 3 ;
et/ou
a+x = 3 ou 4 ;
et
au moins l'une des variables de la formule (Ib), à moins d'indication contraire, indépendamment des autres variables, a la signification suivante :
R⁷= un groupe alkylène en C₁-C₁₀ ou arylène en C₆-C₁₄, ces radicaux pouvant être interrompus par des atomes d'oxygène ou de soufre ou par des groupes -CO-O- ou -O-CO-, ou pouvant porter ces atomes ou groupes en bout de chaîne ;
R⁸ = un groupe alkylène en C₁-C₁₀ ou arylène en C₆-C₁₄, ces radicaux pouvant être interrompus par des atomes d'oxygène ou de soufre ou par des groupes -CO-O- ou -O-CO-, ou pouvant porter ces atomes ou groupes en bout de chaîne ;
X = OCH₃, OC₂H₅ ou Cl ;
R⁹= un groupe alkyle en C₁-C₁₀ ;
et/ou
h = 2 ou 3.

3. Utilisation selon la revendication 1 ou 2, **caractérisée en ce que**
a = 1 et b = 1
et par conséquent le norbornènesilane correspond à la formule générale (IIa) 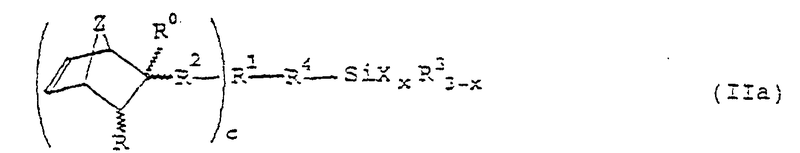 et **en ce que**
g = 1
et par conséquent le mercaptosilane correspond à la formule générale (IIb) :
(HS-R⁷)_{f}R⁸-SiXₕR⁹ ₃₋ₕ (IIb).

4. Utilisation selon la revendication 1 ou 2, **caractérisée en ce que**
a = 1 et c = 1
ce par quoi le norbornènesilane correspond à la formule générale (IIIa) : 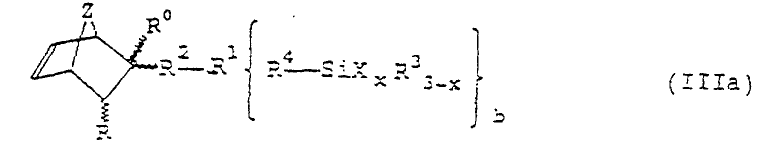 et **en ce que**
f = 1 et g = 1
ce par quoi le mercaptosilane correspond à la formule générale (IIIb) :
HS-R⁷-R⁸-SiXₕR⁹ ₃₋ₕ (IIIb).

5. Utilisation selon la revendication 1 ou 2, **caractérisée en ce que**
b = 1 et c = 1
ce par quoi le norbornènesilane correspond à la formule générale (IVa) 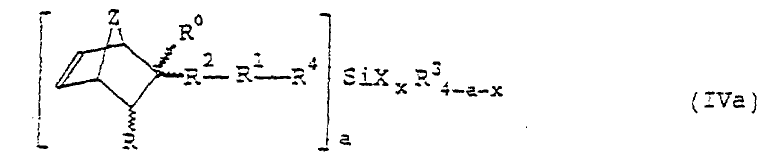 et **en ce que**
f = 1
ce par quoi le mercaptosilane correspond à la formule générale (IVb) :
[HS-R⁷-R⁸]_{g}SiXₕR⁹ _{4-g-h} (IVb).

6. Utilisation selon la revendication 1 ou 2, **caractérisée en ce que**
a = 1 et b = 1 et c = 1
ce par quoi le norbornènesilane correspond à la formule générale (Va)

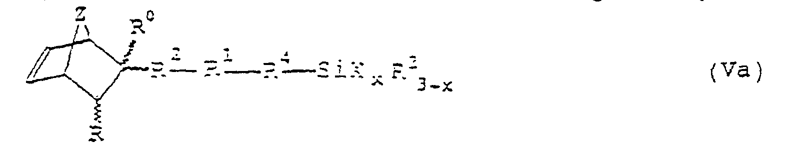

7. Utilisation selon l'une quelconque des revendications 1 à 6, **caractérisée en ce que** la composition contient d'autres composés condensables par hydrolyse.

8. Utilisation selon la revendication 7, **caractérisée en ce qu'**on utilise comme autres composés condensables par hydrolyse au moins un silane de formule générale (VIII)
R¹⁰ ₖ(Z'R¹¹)ₘSiX'₄₋₍ₖ₊ₘ₎ (VIII)
dans laquelle R¹⁰, Z', R¹¹, X', k et m, à moins d'indication contraire, ont indépendamment les uns des autres les significations suivantes :
R¹⁰ = un groupe alkyle en C₁-C₈, alcényle en C₁-C₁₂ ou aryle en C₆-C₁₄,
R¹¹ = un groupe alkylène en C₁-C₈, alcénylène en C₁-C₁₂ ou arylène en C₆-C₁₄,
X' = H, un atome d'halogène, OH, un groupe alcoxy en C₁-C₈,
Z' = le groupe mercapto, carboxy, acryle, méthacryle, allyle, vinyle ou éther vinylique ;
k = 0, 1, 2 ou 3,
m = 0, 1, 2 ou 3 et
k+m = 1, 2 ou 3.

9. Utilisation selon la revendication 7 ou 8, **caractérisée en ce qu'**on utilise comme autres composés condensables par hydrolyse au moins un composé contenant de l'aluminium, du titane ou du zirconium, de formule
AlR¹² ₃ ou MX"_{y}R¹³ _{z}
M, R¹², R¹³, X", y et z ayant, indépendamment les uns des autres, les significations suivantes :
M = Ti ou Zr,
R¹² = un atome d'halogène, OH, un groupe alcoxy en C₁-C₈,
R¹³ = R tel que défini dans la revendication 1,
X" = un atome d'halogène, OH ou un groupe alcoxy en C₁-C₈,
y = 1 à 4, en particulier 2 à 4, et
z = 0 à 3, en particulier 0 à 2.

10. Utilisation selon l'une quelconque des revendications 1 à 9, **caractérisée en ce que** la composition contient le produit de condensation de type acide silicique du norbornènesilane ou le produit de condensation de type acide silicique du mercaptosilane chacun en association avec au moins un partenaire réactionnel pour une polymérisation de thiol-ène.

11. Utilisation selon la revendication 10, **caractérisée en ce que**
(a) on utilise le mercaptosilane, un produit de condensation de type acide silicique du mercaptosilane et/ou un composé de type oligo- ou poly(thiol) en tant que partenaire réactionnel du produit de condensation de type acide silicique du norbornènesilane, et
(b) on utilise le norbornènesilane, un produit de condensation de type acide silicique du norbornènesilane et/ou un composé de type oligo- ou poly(norbornène) en tant que partenaire réactionnel du produit de condensation de type acide silicique du mercaptosilane.

12. Utilisation selon la revendication 11, **caractérisée en ce qu'**on utilise
(a) comme composé oligo- ou poly(thiol) l'o-, p- ou m-dimercaptobenzène ou des esters de l'acide thioglycol- ou 3-mercaptopropionique avec des polyols linéaires ou ramifiés en C₁-C₁₈, et
(b) comme composé oligo- ou polynorbornène, des produits d'addition de Diels-Alder du cyclopentadiène avec des di- ou multi(méth)acrylates, des esters ou uréthannes du 5-norbornène-2-méthanol ou 5-norbornène-2-ol avec des acides di- ou polycarboxyliques ou des di- ou polyisocyanates.

13. Utilisation selon l'une quelconque des revendications 1 à 12, **caractérisée en ce que** la composition contient un initiateur de polymérisation, et en particulier un initiateur pour la polymérisation de thiol-ène.

14. Utilisation selon l'une quelconque des revendications 1 à 13, **caractérisée en ce que** la composition est utilisée sous forme au moins en partie polymérisée.

15. Utilisation selon l'une quelconque des revendications 1 à 14, **caractérisée en ce que** la composition contient
(a) de 5 à 80, plus particulièrement de 10 à 60 %, en poids par rapport à la composition, de produit de condensation de type acide silicique du mercaptosilane ou de produit de condensation de type acide silicique du norbornènesilane, ainsi que
(b) de 0 à 50, plus particulièrement de 0 à 30 %, en poids par rapport à la composition, d'autres composés condensables par hydrolyse, éventuellement sous forme de produits de condensation de type acide silicique de ceux-ci,
(c) de 5 à 80, plus particulièrement de 20 à 70 %, en poids par rapport à la composition, d'un partenaire réactionnel pour une polymérisation de thiol-ène,
(d) de 0,1 à 5, plus particulièrement de 0,2 à 2 %, en poids par rapport à la composition, d'un initiateur de polymérisation,
et/ou
(e) de 0 à 90 % en poids, plus particulièrement de 0 à 80 %, en poids par rapport à la composition, de charges.

16. Utilisation selon l'une quelconque des revendications 1 à 15, **caractérisée en ce que** la composition est utilisée en tant que colle dentaire, matériau de plombage dentaire ou agent de collage pour matériau de plombage dentaire.

17. Utilisation selon l'une quelconque des revendications 1 à 16, **caractérisée en ce que** la composition est appliquée sur la zone à traiter d'une dent naturelle ou artificielle et est soumise à une polymérisation de thiol-ène.

18. Matériau dentaire, **caractérisé en ce qu'**il contient la composition définie dans les revendications 1 à 15.
